# FASCICULE DE BREVET EUROPEEN

(11) **EP 3 731 890 B1**
(45) Date de publication et mention de la délivrance du brevet: **01.05.2024**
(21) Numéro de dépôt: 18842792.6
(22) Date de dépôt: 21.12.2018
(51) Int. Cl.: A61M 1/02, A61M 1/38, A61M 1/36

(54) **SYSTÈME ET PROCÉDÉ DE TRAITEMENT DE LIQUIDE HÉMORRAGIQUE POUR DE L'AUTOTRANSFUSION**
SYSTEM UND VERFAHREN ZUR BEHANDLUNG VON HÄMORRHAGISCHER FLÜSSIGKEIT FÜR AUTOTRANSFUSION
SYSTEM AND METHOD FOR TREATING HEMORRHAGIC FLUID FOR AUTOTRANSFUSION

(30) Priorité: 28.12.2017 FR 1763310; 28.12.2017 FR 1763308
(43) Date de publication de la demande: 04.11.2020
(73) Titulaire: I-Sep, 44200 Nantes (FR)
(72) Inventeur: GADRAT, Francis, 33200 Bordeaux (FR); CHOLLET, Stéphane, 44240 La Chapelle Sur Erdre (FR); PICOT, Sylvain, 69300 Caluire Et Cuire (FR); FOREST-VILLEGAS, Patricia, 69740 Genas (FR)
(74) Mandataire: Regimbeau
(86) Numéro de dépôt international: PCT/FR2018/053501
(87) Numéro de publication internationale: WO 2019/129974

(56) Documents cités:
- US-A- 5 215 519
- US-A1- 2003 229 302

## Description

### DOMAINE DE L'INVENTION

La présente invention concerne le domaine du traitement de liquide hémorragique tel que le sang pour réaliser une autotransfusion chez un patient, notamment au cours d'une intervention chirurgicale.

### ETAT DE LA TECHNIQUE

L'autotransfusion ou transfusion autologue (voir par exemple US 5 215 519 A, US 2003/229302 A1), à savoir la transfusion chez un patient de son propre sang, est de plus en plus pratiquée lors d'interventions chirurgicales puisqu'elle évite les incompatibilités qui peuvent exister avec des transfusions homologues ou allogénique, c'est-à-dire des transfusions à partir d'un sang d'une autre personne, et elle empêche la transmission de maladies infectieuses notamment.

Dans le cas de l'autotransfusion peropératoire, il convient de pouvoir transfuser le sang recueilli directement chez le patient, presque en continu c'est-à-dire en limitant les temps morts dus notamment au traitement du sang, ce traitement se faisant avec un dispositif du traitement indépendamment du patient. Or, lors du recueil, ceci de façon connue, ce sang déjà dilué par les conditions opératoires doit également être additionné d'agents anti-coagulants pour permettre le traitement par un dispositif d'autotransfusion et préserver sa qualité transfusionnelle et les fonctionnalités des éléments sanguins. Ces actions apparaissent nécessaires car en utilisant un liquide vecteur du sang hémorragique recueilli, les globules rouges peuvent être ainsi protégés des traumatismes physiques directs lors du contact mécanique avec les filtres et autres tubulures. Cette dilution dans un liquide vecteur diminue également le contact des globules rouges avec l'air, limitant ainsi fortement leur hémolyse. Enfin, elle permet également de contrôler et d'empêcher l'activité coagulante du sang et éviter la formation de caillots qui ne permettrait pas la récupération des éléments sanguins notamment les globules rouges. Le sang récupéré doit ensuite être transfusé au patient afin de compenser la perte de masse sanguine, mais on se heurte à des problèmes importants. En effet, dans le cas de transfusion de volumes de sang trop dilués, on peut provoquer des phénomènes d'hypervolémie par ces volumes liquidiens transfusés trop importants et des syndromes d'hypocoagulabilité pour les mêmes raisons et/ou par un volume transfusé d'anti-coagulant trop important si non nettoyé.

De plus, lors de l'autotransfusion du sang directement prélevé et seulement anticoagulé et hémodilué, il est possible de transfuser des substances biologiques activées ou dégradées et susceptibles de provoquer des effets secondaires. On peut trouver par exemple des histamines, des kallicréines ou des kinines, des facteurs plasmatiques plus ou moins dégradés dont il vaut mieux se débarrasser ou encore des petites protéines et autres débris cellulaires issus des traumatismes cellulaires.

Pour les conditions d'autotransfusion peropératoire, le traitement du sang consiste donc à recueillir le sang, à l'anticoaguler (ce qui entraîne une dilution) simultanément au prélèvement, puis à préfiltrer dans le bocal de préfiltration et à traiter par le dispositif de traitement de façon à séparer la phase liquidienne de la phase contenant les éléments cellulaires permettant ainsi d'une part une concentration de la phase recueillie et destinée à être transfusée et d'autre part une collecte de la phase liquidienne à éliminer. Il convient de préciser que ces étapes doivent être faites le plus rapidement possible puisque le patient, dans des conditions peropératoires, a généralement besoin d'être transfusé en urgence.

Il a été développé différentes techniques plus ou moins complexes et efficaces pour réaliser de l'autotransfusion chez un patient pendant les interventions chirurgicales.

Il existe par exemple des systèmes d'autotransfusion basés sur des techniques de centrifugation. La centrifugation du sang prélevé en autotransfusion réalise la séparation des globules rouges (GR) et du plasma contenant les plaquettes (PRP) et les protéines. Un fort taux de recouvrement des plaquettes est donc impossible par cette méthode.

En outre, pour que le processus soit rapide, il peut être effectué une centrifugation renforcée mais une couche va alors se former à l'interface entre les globules rouges et le plasma, que l'on appelle couche leuco plaquettaire qui est un mélange de plaquettes et de globules blancs (GB). Cette couche est donc impropre à une transfusion directe.

Il est donc nécessaire d'appliquer des traitements complémentaires pour récupérer les plaquettes et éliminer les éléments non désirés tels que la couche leuco plaquettaire. En outre, lorsque la centrifugation est trop forte, il va y avoir une élimination des plaquettes et donc un appauvrissement de la qualité du concentrât à transfuser.

Le prélèvement sous aspiration du sang et fluides biologiques par le chirurgien induit un traumatisme sur les globules rouges entraînant une hémolyse mécanique des plus fragiles. La centrifugation, mécanisme connu et largement utilisé en autotransfusion, entraîne également une légère hémolyse mécanique. Lors du traitement du sang par centrifugation en peropératoire, les globules rouges subissent donc des traumatismes entraînant une hémolyse importante, qui peut aller jusqu'à 19%. En mode urgence (centrifugation renforcée pour diminuer le temps de traitement), ce taux d'hémolyse peut s'élever à 33%.

Une alternative aux procédés actuels de traitement du sang en peropératoire par centrifugation est souhaitée car la centrifugation, outre les problèmes d'hémolyse, élimine la majorité des plaquettes (taux de recouvrement inférieur à 10%). Cette perte directe de cellules d'intérêt, participant directement à l'hémostase primaire (agrégation plaquettaire au niveau de la plaie), est problématique lors d'une opération comme on peut aisément le comprendre. C'est pourquoi, lorsque les pertes sont trop importantes, les médecins ont recours à la transfusion de produits sanguins labiles dont un ou plusieurs concentrés plaquettaires homologues. Un procédé qui permettrait de conserver et de transfuser les plaquettes (avec de préférence un taux de recouvrement supérieur à 50%) du patient serait par conséquent appréciable.

Il a ainsi été développé des systèmes d'autotransfusion alternatifs, basés sur des dispositifs de filtration membranaire. Cela est par exemple le cas des systèmes d'autotransfusion présentés dans le brevet US 4,886,487, dans le brevet US 5,215,519 et dans la demande de brevet US 2003/229302. Ces systèmes sont avantageux dans le sens où ils permettent une réelle séparation des éléments non désirés pour la transfusion du sang, sans éliminer les éléments importants comme les plaquettes comme cela est le cas lors d'une centrifugation. De tels systèmes présentent toutefois un certain nombre d'inconvénients, notamment en termes d'efficacité.

Les cellules (GR, GB et plaquettes) présentent une déformabilité membranaire importante leurs permettant de passer à travers des micro-vaisseaux sanguins ou les plaies. Néanmoins, lors de filtration sur membrane, une baisse du flux de filtration est constatée tout au long du processus. Le déclin du flux peut être expliqué par plusieurs facteurs, à savoir, l'adsorption, l'entrave stérique, les effets de viscosité, l'obstruction et le colmatage des pores, ainsi que le gradient de concentration à l'interface membrane/solution.

Dans le cas d'une filtration, la taille des pores et l'hydrophilie des matériaux doivent être contrôlées afin de laisser passer ou non ces cellules. Dans le cas d'une utilisation d'une membrane à pores de diamètres inférieurs à 10 µm, en particulier inférieurs à 1 µm, le colmatage des membranes par les cellules est obligatoire en filtration frontale, d'où la nécessité d'une filtration tangentielle. Les plaquettes ont également un fort pouvoir d'adhésion après activation et ont tendance à s'adsorber en surface des membranes ou aux protéines plasmatiques et colmater les membranes.

La filtration tangentielle est soumise à une quantité de matière pouvant passer à travers la membrane par unité de temps, ce qui limite en général la vitesse de traitement. On parle de débit transmembranaire ou de coefficient de filtration. Néanmoins, le patient subissant une hémorragie massive ne peut voir augmenter son coefficient de perte de chance en raison d'un temps de filtration supérieur. Il est donc primordial que le temps de traitement du sang en peropératoire par filtration soit comparable à la méthode classique de centrifugation, où une séparation des globules rouges et du plasma peut être faite dans un temps rapide de 4-6 minutes pour un volume de sang collecté de 500 ml.

De même que pour le temps de traitement, les performances du traitement par filtration membranaire doivent être au moins comparables au traitement du sang en peropératoire par la méthode de centrifugation (taux de recouvrement de GR supérieur à 80%, quantité d'héparine inférieure à 0.5 UI/ml).

Il existe donc aujourd'hui un besoin d'un système de traitement de sang pour autotransfusion amélioré, permettant notamment de résoudre au moins l'un des inconvénients précités.

Un but de la présente invention est aussi de proposer un système de traitement de sang pour autotransfusion qui est facile d'utilisation, et intuitif, de manière à pouvoir être utilisé par des professionnels avec peu ou pas de formation.

### EXPOSE DE L'INVENTION

A cette fin, on propose un système (ne correspondant pas à l'invention revendiquée) de traitement d'un liquide hémorragique préalablement prélevé un patient en vue d'une autotransfusion, incluant une unité de traitement du liquide hémorragique, ladite unité de traitement comprenant :
- un dispositif de filtration pour filtration tangentielle comprenant une membrane de filtration agencée dans un boitier de manière à séparer une chambre d'admission d'une chambre d'évacuation, la chambre d'admission et la chambre d'évacuation ayant chacune une entrée et une sortie de fluides ;
- une poche de traitement ayant une entrée et une sortie reliées fluidiquement par une ligne de recirculation à la sortie et à l'entrée de la chambre d'admission du dispositif de filtration, respectivement, permettant une circulation du liquide hémorragique dans la ligne de recirculation dans un sens allant de la sortie de la poche de traitement vers l'entrée de la poche de traitement à travers la chambre d'admission du dispositif de filtration ;
- une ligne d'admission reliée fluidiquement à la ligne de recirculation entre la sortie de la poche de traitement et l'entrée de la chambre d'admission du dispositif de filtration permettant d'alimenter l'unité de traitement avec le liquide hémorragique prélevé en vue d'une filtration à travers la membrane de filtration du dispositif de filtration afin de retirer du liquide hémorragique un filtrat comprenant des composés non désirés pour l'autotransfusion ;
- une ligne de transfusion reliée fluidiquement à la ligne de recirculation entre la sortie de la poche de traitement et l'entrée de la chambre d'admission du dispositif de filtration permettant de récupérer le liquide hémorragique traité contenu dans ladite poche de traitement ;
- une ligne d'évacuation reliée fluidiquement à la sortie de la chambre d'évacuation du dispositif de filtration de sorte à évacuer le filtrat ayant traversé la membrane de filtration depuis la chambre d'admission ;
caractérisé en ce que l'unité de traitement comprend en outre
- une ligne de nettoyage reliée fluidiquement à l'entrée de la chambre d'évacuation du dispositif de filtration pour amener un liquide de nettoyage dans ladite chambre d'évacuation ; et
- un premier organe de régulation de flux agencé pour une régulation de flux dans la ligne de nettoyage et un deuxième organe de régulation de flux agencé pour une régulation de flux dans la ligne d'évacuation de manière à pouvoir contrôler la pression de liquide de nettoyage dans la chambre d'évacuation.

Des aspects préférés mais non limitatifs de ce système de traitement, pris seuls ou en combinaison, sont les suivants :
- la ligne de nettoyage est en outre reliée fluidiquement à la ligne de recirculation à une première position entre la sortie de la poche de traitement et l'entrée de la chambre d'admission du dispositif de filtration, l'unité de traitement comprenant en outre une ligne de dilution destinée à amener un liquide de dilution dans l'unité de traitement, la ligne de dilution étant reliée fluidiquement à la ligne de recirculation à une deuxième position entre la sortie de la poche de traitement et l'entrée de la chambre d'admission du dispositif de filtration, le liquide de dilution pouvant être utilisé en tant que liquide de nettoyage.
- l'unité de traitement comprend un troisième organe de régulation de flux agencé pour une régulation de flux dans la ligne de dilution, un quatrième organe de régulation de flux agencé pour une régulation de flux dans la ligne de recirculation à la sortie de la poche de traitement, et un cinquième organe de régulation de flux agencé pour une régulation de flux dans la ligne de recirculation à l'entrée de la chambre d'admission du dispositif de filtration.
- la deuxième position est située en amont de la première position dans le sens de circulation du fluide dans la ligne de recirculation lors du traitement du liquide hémorragique.
- le système comprend une unique pompe péristaltique agencée de manière à faire circuler le liquide hémorragique dans la ligne de recirculation dans un sens allant de la sortie de la poche de traitement vers l'entrée de la poche de traitement à travers la chambre d'admission du dispositif de filtration, ladite pompe péristaltique étant positionnée dans la ligne de recirculation entre la sortie de la poche de traitement et l'entrée de la chambre d'admission du dispositif de filtration entre la deuxième position et la première position.
- le système comprend au moins une pompe péristaltique agencée de manière à faire circuler le liquide hémorragique dans la ligne de recirculation dans un sens allant de la sortie de la poche de traitement vers l'entrée de la poche de traitement à travers la chambre d'admission du dispositif de filtration.
- la poche de traitement comprend un dispositif séparateur pouvant être actionné pour séparer la poche de traitement en une première chambre de traitement du côté de l'entrée de la poche de traitement et une deuxième chambre de traitement du côté de la sortie de la poche de traitement.
- la poche de traitement a une forme sensiblement parallélépipédique avec l'entrée et la sortie de part et d'autre de la poche de traitement selon une diagonale, la proche de traitement présentant en outre une cavité interne ayant une forme rétrécie du côté de la sortie.
- la membrane de filtration du dispositif de filtration est une membrane de filtration à fibres creuses, lesdites fibres creuses formant la membrane de filtration s'étendant longitudinalement dans le boitier.
- la membrane de filtration à fibres creuses du dispositif de filtration comprend des fibres creuses formées à partir d'un mélange de polyester sulfone et de polyvinyle pyrrolidone.
- la membrane de filtration du dispositif de filtration a une porosité globale comprise entre 0,1 µm et 1 µm, de préférence de l'ordre de 0,6 µm.
- la membrane de filtration du dispositif de filtration a une surface de filtration globale comprise entre 0,1 m² et 1 m², et de préférence comprise entre 0,2 m² et 0,6 m².
- l'unité de traitement comprend un sixième organe de régulation de flux agencé pour une régulation de flux dans la ligne de transfusion.
- le système de traitement comprend une pluralité de valves de régulation, chaque valve de régulation étant respectivement destinée à coopérer avec l'un des organes de régulation afin de réguler le flux correspondant.
- l'unité de traitement comprend un gabarit permettant une fixation de la ligne d'admission, la ligne d'évacuation, la ligne de recirculation, la ligne de transfusion et la ligne de nettoyage.
- le système de traitement comprend une unité de support, le gabarit de l'unité de traitement ayant une forme en détrompeur permettant de coupler l'unité de traitement à l'unité de support selon un unique positionnement, de préférence de manière amovible.
- l'unité de support forme un plan de support horizontal, le dispositif de filtration de l'unité de traitement étant destiné à être couplé à l'unité de support de sorte que les fibres creuses de la membrane de filtration s'étendent selon une direction non comprise dans le plan de support horizontal.
- le système de traitement comprend une unité de transfusion, ladite unité de transfusion comprenant une poche de transfusion ayant une entrée destinée à être reliée à la ligne de transfusion afin de collecter le liquide hémorragique traité en provenance de la poche de traitement avant une transfusion au patient.
- le système de traitement comprend une unité de récupération du filtrat, ladite unité de récupération comprenant une poche de récupération ayant une entrée destinée à être reliée fluidiquement à la ligne d'évacuation, ladite poche de récupération étant en outre destinée à être couplée à un dispositif de mise en dépression de la poche de récupération de manière à faire circuler le filtrat de la chambre d'évacuation du dispositif de filtration vers la poche de récupération à travers la ligne d'évacuation.
- le système de traitement comprend une unité de récupération du filtrat, ladite unité de récupération comprenant une poche de récupération ayant une entrée destinée à être reliée fluidiquement à la ligne d'évacuation, ladite poche de récupération étant en outre agencée par rapport au dispositif de filtration de l'unité de traitement pour créer une dépression de la poche de récupération par rapport au dispositif de filtration de manière à faire circuler le filtrat de la chambre d'évacuation du dispositif de filtration vers la poche de récupération à travers la ligne d'évacuation.
- le système de traitement comprend une unité de collecte du liquide hémorragique comprenant un réceptacle de collecte du liquide hémorragique préalablement prélevé chez le patient, ledit réceptacle de collecte ayant une sortie reliée fluidiquement à la ligne d'admission, ledit réceptacle de collecte intégrant de préférence un dispositif de préfiltration permettant de faire une préfiltration du liquide hémorragique avant d'être transmis dans l'unité de traitement.
- le système de traitement comprend en outre un dispositif de préfiltration supplémentaire placé dans la ligne d'admission.

On propose également un procédé (ne correspondant pas à l'invention revendiquée) d'utilisation de ce système de traitement de liquide hémorragique préalablement prélevé chez un patient en vue d'une autotransfusion ultérieure dans lequel, après un traitement partiel ou total du liquide hémorragique avec le dispositif de filtration, on effectue un nettoyage de la membrane de filtration en créant un contre-flux transmembranaire, le contre-flux étant créé en obstruant la ligne d'évacuation au niveau du deuxième organe de régulation de flux et en injectant le liquide de nettoyage dans la chambre d'évacuation depuis la ligne de nettoyage, la pression créée dans la chambre d'évacuation par l'injection du liquide de nettoyage créant un contre-flux à travers la membrane de filtration permettant de décoller tout ou partie des éléments retenus sur la membrane de filtration.

Des aspects préférés mais non limitatifs de ce procédé d'utilisation du système de traitement, pris seuls ou en combinaison, sont les suivants :
- le nettoyage à contre-flux est effectué à intervalles réguliers pendant le traitement d'un volume déterminé de liquide hémorragique.
- le nettoyage à contre-flux est effectué après le traitement total d'un volume déterminé de liquide hémorragique.
- le nettoyage à contre-flux est effectué en faisant varier la vitesse de circulation du liquide de nettoyage, en particulier par augmentation et diminution de ladite vitesse de circulation du liquide de nettoyage.
- on traite un volume déterminé de liquide hémorragique issu de la ligne d'admission en le faisant circuler dans la ligne de circulation afin de traverser à plusieurs reprises le dispositif de filtration pour retirer les composés non désirés pour l'autotransfusion, la poche de traitement permettant de maintenir un flux ayant un débit continu dans la ligne de circulation quel que soit le volume de liquide hémorragique à traiter.
- pendant le traitement d'un volume déterminé de liquide hémorragique, on obstrue la sortie de la poche de traitement, puis on injecte dans la ligne de circulation un liquide de dilution destiné à traverser le dispositif de filtration de manière à éliminer le liquide hémorragique présent dans la ligne de circulation, puis on isole le liquide hémorragique traité présent dans la poche de traitement lorsque le liquide présent dans la ligne de circulation a un taux d'hématocrite inférieur à une valeur seuil.

Selon un autre aspect préféré mais non limitatif de ce procédé d'utilisation du système de traitement, pris seul ou en combinaison avec les aspects précédents, on effectue - avant et/ou après le nettoyage à contre-flux - un nettoyage de la membrane de filtration par rinçage, le rinçage étant opéré en obstruant la sortie de la poche de traitement, en obstruant la ligne d'évacuation au niveau de la sortie de la chambre d'évacuation du dispositif de filtration, et en injectant dans la chambre d'admission un liquide de dilution destiné à traverser le dispositif de filtration.

On propose par ailleurs un système de traitement d'un liquide hémorragique préalablement prélevé chez un patient en vue d'une autotransfusion, incluant une unité de traitement du liquide hémorragique, ladite unité de traitement comprenant :
- un dispositif de filtration comprenant une membrane de filtration pour filtration tangentielle agencée dans un boitier de manière à séparer une chambre d'admission d'une chambre d'évacuation, la chambre d'admission et la chambre d'évacuation ayant chacune une entrée et une sortie de fluides ;
- une poche de traitement ayant une entrée et une sortie reliées fluidiquement par une ligne de recirculation à la sortie et à l'entrée de la chambre d'admission du dispositif de filtration, respectivement, permettant une circulation du liquide hémorragique dans la ligne de recirculation dans un sens allant de la sortie de la poche de traitement vers l'entrée de la poche de traitement à travers la chambre d'admission du dispositif de filtration ;

- une ligne d'admission reliée fluidiquement à la ligne de recirculation entre la sortie de la poche de traitement et l'entrée de la chambre d'admission du dispositif de filtration permettant d'alimenter l'unité de traitement avec le liquide hémorragique prélevé en vue d'une filtration à travers la membrane de filtration du dispositif de filtration afin de retirer du liquide hémorragique un filtrat comprenant des composés non désirés pour l'autotransfusion ;
- une ligne de transfusion reliée fluidiquement à la ligne de recirculation entre la sortie de la poche de traitement et l'entrée de la chambre d'admission du dispositif de filtration permettant de récupérer le liquide hémorragique traité contenu dans ladite poche de traitement ;
- une ligne d'évacuation reliée fluidiquement à la sortie de la chambre d'évacuation du dispositif de filtration de sorte à évacuer le filtrat ayant traversé la membrane de filtration depuis la chambre d'admission ;
caractérisé en ce que l'unité de traitement comprend en outre
- un premier organe de régulation de flux agencé pour une régulation de flux dans la ligne de recirculation à la sortie de la poche de traitement, et
- une ligne de dilution destinée à amener un liquide de dilution dans l'unité de traitement, la ligne de dilution étant reliée fluidiquement à la ligne de recirculation à une position entre la sortie de la poche de traitement et l'entrée de la chambre d'admission du dispositif de filtration.

Des aspects préférés mais non limitatifs de ce système de traitement, pris seuls ou en combinaison, sont les suivants :
- un capteur optique est agencé au niveau de l'entrée de la poche de traitement pour détecter la nature du liquide arrivant au niveau de l'entrée.
- la poche de traitement comprend un dispositif séparateur pouvant être actionné pour séparer la poche de traitement en une première chambre de traitement du côté de l'entrée de la poche de traitement et une deuxième chambre de traitement du côté de la sortie de la poche de traitement.
- l'unité de traitement comprend un deuxième organe de régulation de flux agencé pour une régulation de flux dans la ligne de dilution, et un troisième organe de régulation de flux agencé pour une régulation de flux dans la ligne de recirculation à l'entrée de la chambre d'admission du dispositif de filtration.
- la poche de traitement a une forme sensiblement parallélépipédique avec l'entrée et la sortie de part et d'autre de la poche de traitement selon une diagonale, la proche de traitement présentant en outre une cavité interne ayant une forme rétrécie du côté de la sortie.
- la membrane de filtration du dispositif de filtration est une membrane de filtration à fibres creuses, lesdites fibres creuses formant la membrane de filtration s'étendant longitudinalement dans le boitier.
- la membrane de filtration à fibres creuses du dispositif de filtration comprend des fibres creuses formées à partir d'un mélange de polyester sulfone et de polyvinyle pyrrolidone.
- la membrane de filtration du dispositif de filtration a une porosité globale comprise entre 0,1 µm et 1 µm, de préférence de l'ordre de 0,6 µm.
- la membrane de filtration du dispositif de filtration a une surface de filtration globale comprise entre 0,1 m² et 1 m², et de préférence comprise entre 0,2 m² et 0,6 m².
- le système de traitement comprend au moins une pompe péristaltique agencée de manière à faire circuler le liquide hémorragique dans la ligne de recirculation dans un sens allant de la sortie de la poche de traitement vers l'entrée de la poche de traitement à travers la chambre d'admission du dispositif de filtration.
- le système de traitement comprend une pluralité de valves de régulation, chaque valve de régulation étant respectivement destinée à coopérer avec l'un des organes de régulation afin de réguler le flux correspondant.
- l'unité de traitement comprend un gabarit permettant une fixation de la ligne d'admission, la ligne d'évacuation, la ligne de recirculation, la ligne de dilution, et la ligne de transfusion.
- le système de traitement comprend une unité de support, le gabarit de l'unité de traitement ayant une forme en détrompeur permettant de coupler l'unité de traitement à l'unité de support selon un unique positionnement.
- l'unité de support forme un plan de support horizontal, le dispositif de filtration de l'unité de traitement étant destiné à être couplé à l'unité de support de sorte que des fibres creuses de la membrane de filtration s'étendent selon une direction non comprise dans le plan de support horizontal.
- le système de traitement comprend une unité de transfusion, ladite unité de transfusion comprenant une poche de transfusion ayant une entrée destinée à être reliée à la ligne de transfusion afin de collecter le liquide hémorragique traité en provenance de la poche de traitement avant une transfusion au patient.
- le système de traitement comprend une unité de récupération du filtrat, ladite unité de récupération comprenant une poche de récupération ayant une entrée destinée à être reliée fluidiquement à la ligne d'évacuation, ladite poche de récupération étant en outre destinée à être couplée à un dispositif de mise en dépression de la poche de récupération de manière à faire circuler le filtrat de la chambre d'évacuation du dispositif de filtration vers la poche de récupération à travers la ligne d'évacuation.
- le système de traitement comprend une unité de récupération du filtrat, ladite unité de récupération comprenant une poche de récupération ayant une entrée destinée à être reliée fluidiquement à la ligne d'évacuation, ladite poche de récupération étant en outre agencée par rapport au dispositif de filtration de l'unité de traitement pour créer une dépression de la poche de récupération par rapport au dispositif de filtration de manière à faire circuler le filtrat de la chambre d'évacuation du dispositif de filtration vers la poche de récupération à travers la ligne d'évacuation.
- le système de traitement comprend une unité de collecte du liquide hémorragique comprenant un réceptacle de collecte du liquide hémorragique préalablement prélevé chez le patient, ledit réceptacle de collecte ayant une sortie reliée fluidiquement à la ligne d'admission, ledit réceptacle de collecte intégrant de préférence un dispositif de préfiltration permettant de faire une préfiltration du liquide hémorragique avant d'être transmis dans l'unité de traitement.
- le système de traitement comprend en outre un dispositif de préfiltration supplémentaire placé dans la ligne d'admission.

On propose également un procédé d'utilisation de ce système de traitement de liquide hémorragique préalablement prélevé chez un patient en vue d'une autotransfusion ultérieure dans lequel, après un traitement partiel ou total du liquide hémorragique avec le dispositif de filtration, on injecte un liquide de dilution depuis la ligne de dilution dans la ligne de circulation afin de traverser le dispositif de filtration.

Selon un aspect préféré de ce procédé d'utilisation du système de traitement, on effectue un nettoyage de la membrane de filtration par rinçage, le rinçage étant opéré en obstruant la sortie de la poche de traitement au niveau du premier organe de régulation de flux, en obstruant la ligne d'évacuation au niveau du deuxième organe de régulation de flux et en injectant le liquide de nettoyage dans la chambre d'admission depuis la ligne de dilution.

Dans ce cas, le rinçage peut être arrêté dès que le capteur optique détecte la présence du liquide de dilution.

De manière préférée, avant le rinçage, on peut commander le dispositif séparateur pour isoler le liquide hémorragique traité dans la deuxième chambre de traitement.

Selon un autre aspect préféré de ce procédé d'utilisation du système de traitement, on effectue, de façon complémentaire ou alternative, une dilution du liquide hémorragique contenu dans le système de traitement , la dilution étant opérée en obstruant la sortie de la poche de traitement au niveau du premier organe de régulation de flux, puis en injectant le liquide de nettoyage dans la chambre d'admission depuis la ligne de dilution, puis en isolant le liquide hémorragique traité présent dans la poche de traitement lorsque le liquide présent dans la ligne de circulation a un taux d'hématocrite inférieur à une valeur seuil.

### DESCRIPTION DES FIGURES

D'autres caractéristiques et avantages de l'invention ressortiront encore de la description qui suit, laquelle est purement illustrative et non limitative et doit être lue en regard des dessins annexés, sur lesquels :
- la figure 1 est une vue en perspective schématique d'un système de traitement selon l'invention ;
- la figure 2 est une représentation schématique d'un premier agencement des connexions fluidiques pour le système de traitement selon l'invention ;
- la figure 3 est une représentation d'une unité de traitement d'un système de traitement, selon un premier mode de réalisation du premier agencement ;
- la figure 4 est une représentation d'une unité de traitement d'un système de traitement, selon un deuxième mode de réalisation du premier agencement ;
- la figure 5 est une représentation schématique d'une poche de traitement pour l'unité de traitement d'un système de traitement selon l'invention ;
- la figure 6 est une représentation schématique d'un deuxième agencement des connexions fluidiques pour le système de traitement selon l'invention ;
- la figure 7 est une représentation schématique illustrant un exemple de positionnement d'un dispositif de préfiltration supplémentaire pour le système de traitement présenté.

### DESCRIPTION DETAILLEE DE L'INVENTION

### Description du système de traitement d'un liquide hémorragique en vue d'une autotransfusion

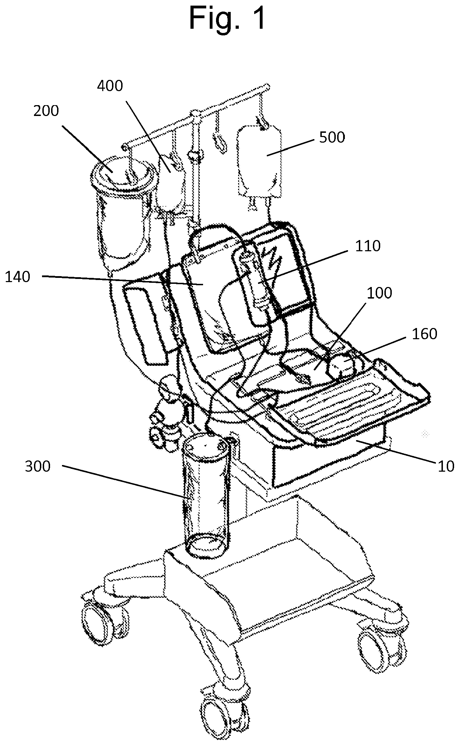

La figure 1 illustre un exemple non-limitatif d'un système de traitement d'un liquide hémorragique, notamment le sang, d'un patient en vue notamment d'une autotransfusion.

Le système de traitement proposé comporte un certain nombre d'unités fonctionnelles, amovibles les unes par rapport aux autres pour en faciliter l'usage par les praticiens, notamment au cours d'une intervention chirurgicale.

L'une des particularités du système de traitement proposé réside dans l'unité de traitement 100 du sang qui va être décrite en détail plus loin.

Le système de traitement proposé comprend de préférence une unité de support 10 qui peut se présenter sous diverses formes, avec notamment comme représenté à la figure 1 un corps principal de support pouvant être monté sur roulettes. De préférence, l'unité de support 10 intègre les éléments non-consommables du système de traitement, c'est-à-dire pouvant être réutilisés pendant plusieurs cycles de traitement successifs, en particulier les éléments du système n'étant pas en contact direct avec le liquide hémorragique du patient ou toute autre substance qui pourrait entrainer des contaminations.

L'unité de support 10 peut notamment intégrer des moyens de traitement de données, sous la forme d'un ou plusieurs processeurs par exemple, mais également des moyens de commande permettant de piloter le traitement du liquide hémorragique en fonction de paramètres de traitement prédéterminés et/ou en fonction d'informations de commande entrées par l'utilisateur du système. A cet égard, l'unité de support 10 peut inclure des moyens de saisie des informations de commande, tel qu'un clavier, des actionneurs tactiles, un système de reconnaissance vocal ou autres. De préférence, il est prévu également des moyens de diffusion d'informations pour informer l'utilisateur sur le cycle de traitement, ces moyens de diffusion d'information pouvant être visuels, sonores et/ou tactiles, incluant par exemple un écran, des lampes ou diodes lumineuses, un haut-parleur, un vibrateur ou autres.

L'unité de support 10 intègre également de préférence les éléments pour l'alimentation en énergie, notamment électrique, du système de traitement. L'unité de support 10 pourrait intégrer une batterie pour fournir de l'énergie au système, et elle comprend en tout état de cause des connecteurs électriques permettant de raccorder ladite unité de support 10 à une prise électrique, par exemple une prise murale d'un hôpital.

La circulation des liquides dans le système de traitement est réalisée par des moyens d'entrainement de liquide qui sont intégrés dans l'unité de support 10 ou externes.

En tant que moyens d'entrainement des liquides, il peut par exemple être prévu une ou plusieurs pompes péristaltiques 160 permettant de mettre en mouvement les liquides présents dans un circuit de circulation du système de traitement, dans un sens de déplacement du liquide spécifique, mais également possiblement en sens inverse. De préférence, la ou lesdites pompes péristaltiques sont intégrées dans l'unité de support 10, où elles peuvent directement être alimentées électriquement. Les moyens d'entrainement de liquide sont prévus de préférence pour permettre une circulation du liquide hémorragique dans l'unité de traitement 100 avec un débit compris entre 10 ml/min et 4000 ml/min, de préférence entre 100 ml/min à 2000 ml/min, et de préférence encore entre 200 ml/min à 1400 ml/min.

Parmi les moyens d'entrainement des liquides, il peut également être prévus des systèmes de mise sous vide, qui sont branchés au circuit de circulation des liquides pour créer des dépressions favorisant le déplacement du liquide dans le circuit de circulation dans un sens de déplacement spécifique. A cet égard, il pourrait être intégré dans le système de traitement, et plus spécifiquement dans l'unité de support 10, une ou plusieurs pompes à vide pour créer les dépressions requises. Il peut également être prévu que le système de traitement comprenne des connecteurs permettant une connexion avec les prises murales de vide du lieu où le système de traitement est utilisé, et également des régulateurs de vide pour que le vide puisse être contrôlé spécifiquement. De préférence, il est prévu des moyens permettant d'appliquer un vide de 0 à -100 kPa.

L'unité de support 10 peut encore intégrer d'autres éléments pouvant être réutilisés pendant plusieurs cycles de traitement successifs et qui sont liés fonctionnellement aux opérations menées par l'unité de traitement 100 qui va être détaillée plus loin.

Ainsi, l'unité de support peut intégrer des valves de régulation de flux, comme des électrovannes fonctionnant par exemple avec des électroaimants ou des moteurs pas à pas, qui sont agencées pour coopérer avec les tuyaux de l'unité de traitement 100 pour permettre une régulation du flux du liquide circulant dans ladite unité de traitement 100.

Il peut également être prévu des capteurs permettant de suivre l'évolution du traitement au cours d'un cycle de traitement spécifique. De tels capteurs peuvent par exemple inclure des capteurs de pression, des dispositifs de pesées, des capteurs notamment optiques pour calculer l'hématocrite (e.g. pour calculer le taux d'hématocrite du liquide circulant dans l'unité de traitement).

Comme précisé plus haut, ces éléments réutilisables sont de préférence intégrés à l'unité de support 10 mais il pourrait aussi être envisagé que l'unité de traitement 100 en intègre un ou plusieurs.

Le système de traitement comprend en outre une unité de collecte 200 du liquide hémorragique qui est adaptée pour être positionnée sur l'unité de support 10 comme illustré à la figure 1.

Une telle unité de collecte 200 comprend un réceptacle de collecte 210 du liquide hémorragique prélevé chez le patient, par exemple au cours d'une intervention chirurgicale, ou préalablement.

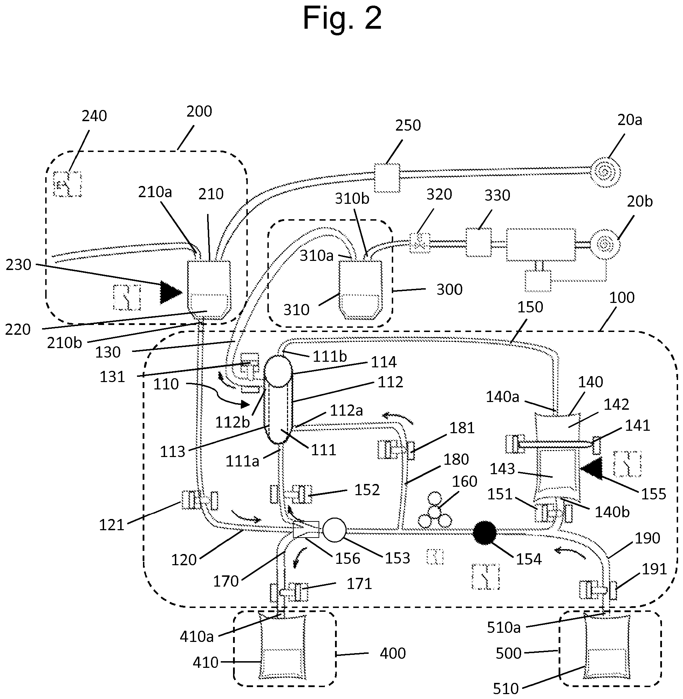

Comme on le voit illustré à la figure 2, ce réceptacle de collecte 210 a une sortie 210b reliée fluidiquement à une ligne d'admission 120 prévue dans l'unité de traitement 100.

Il comprend également une entrée 210a destinée à être couplée à des moyens de prélèvement du sang connus en eux-mêmes, comprenant généralement une canule d'aspiration du liquide hémorragique et des moyens de dosage d'agents diluants et anticoagulants notamment. Une source spécifique de ces agents diluants et/ou anticoagulants, comme par exemple une composition de cristalloïde héparinée, pourrait être connectée directement à l'entrée 210a du réceptacle de collecte 210.

De préférence encore, le réceptacle de collecte 210 intègre un dispositif de préfiltration 220 permettant de faire une préfiltration du liquide hémorragique prélevé avant d'être transmis dans l'unité de traitement 100. Une telle préfiltration est généralement destinée à filtrer les particules de relativement grosses dimensions, pour retirer par exemple caillots sanguins, morceaux d'os voire morceaux de tissus présents dans le liquide hémorragique prélevé.

La préfiltration est notamment effectuée pour retenir les particules ayant une taille supérieure à plusieurs dizaines de micromètres.

On peut par exemple utiliser un dispositif de préfiltration 220 de type filtration frontale ayant un gradient de porosité, pour retenir les particules de tailles décroissantes, le gradient de porosité allant par exemple progressivement de 150 µm à 40 µm.

Ce dispositif de préfiltration 220 peut par exemple être un filtre de type multicouches, plus précisément avec une couche en maille tissée pour retenir les plus gros éléments, une couche en maille non tissée dont l'épaisseur permettra de retenir par encombrement stérique des éléments de moindre taille, et une dernière couche à maille fine tissée pour retenir les plus petits éléments.

De manière préférée, l'unité de collecte 200 est en outre reliée à une source de vide, par exemple à une prise murale de vide 20a via un régulateur de vide 250, pour optimiser la préfiltration avec le dispositif de préfiltration 220.

Il peut en outre être prévu dans l'unité de collecte 200 un système de pesée 230, tel qu'un capteur d'effort formant peson, prévu pour mesurer la quantité de liquide hémorragique collecté présent dans le réceptacle de collecte 210. Ce système de pesée 230 va permettre de donner des informations pour commander le cycle de traitement, permettant par exemple de débuter un cycle de traitement lorsque le réceptacle de collecte 210 contient suffisamment de liquide hémorragique pour débuter un cycle de traitement. Le système de pesée 230 permet également de contrôler la quantité de liquide hémorragique injectée dans l'unité de traitement 100.

Le système de traitement comprend en outre une unité de transfusion 400 qui est destinée à être agencée sur l'unité de support 10 et couplée à la ligne de transfusion 170 de l'unité de traitement 100.

Plus précisément, l'unité de transfusion 400 comprend une poche de transfusion 410 ayant une entrée 410a destinée à être reliée à la ligne de transfusion 170 afin de collecter le liquide hémorragique traité en provenance d'une poche de traitement 140 intégrée dans l'unité de traitement 100, avant une transfusion au patient. Plus précisément, lorsque l'on souhaite effectuer une transfusion au patient, il convient de déconnecter la poche de transfusion 410 du système de traitement et de la connecter au patient pour transfusion du liquide hémorragique traité.

Le système de traitement comprend également une unité de récupération 300 également montée sur l'unité de support 10 et destiné à récupérer le filtrat issu d'unité de traitement 100, c'est-à-dire les déchets prélevés du liquide hémorragique ne convenant pas à une transfusion au patient.

Cette unité de récupération 300 comprend ainsi une poche de récupération 310 ayant une entrée 310a destinée à être reliée fluidiquement à la ligne d'évacuation 130 de l'unité de traitement 100.

Préférentiellement, la poche de récupération 310 est en outre prévue pour être mise en dépression de manière à entrainer le liquide circulant depuis la ligne d'évacuation 130 jusque ladite poche de récupération 310.

A cette fin, la poche de récupération 310 peut être couplée à un dispositif de mise en dépression, comme par exemple un système de mise sous vide utilisant une prise murale de vide 20b et un régulateur de vide 330 et/ou un système autonome comprenant à minima une pompe à vide et un régulateur électronique. Un contrôle fin du vide appliqué permet de maîtriser la dépression appliquée, pour éviter qu'elle ne soit trop faible et que le traitement n'en soit ralenti, ni qu'elle ne soit trop forte ce qui pourrait endommager les globules rouges ou le filtre. Une vanne 320 de coupure et mise à l'air peut également être prévue pour débrayer le système de mise sous vide au besoin.

Alternativement ou en complément, la mise en dépression de la poche de récupération 310 peut être créée par un agencement spécifique par rapport à l'unité de traitement 100, notamment par une différence de hauteur entre les deux unités. Comme on le voit représenté à la figure 1 par exemple, l'unité de récupération 300 est de préférence agencée en partie basse de l'unité de support 10, proche des roues par exemple, tandis que l'unité de traitement 100 est agencée en partie haute, ou à tout le moins à un niveau plus haut que l'unité de récupération 300. Il est à noter qu'une mise en dépression de la poche de récupération 310 par le simple agencement des éléments entre eux, sans utilisation d'un vide artificiel (avec une pompe à vide par exemple) peut être avantageux pour limiter les risques d'hémolyse notamment.

Il peut par exemple être prévue une distance verticale séparant l'unité de traitement 100 de l'unité de récupération 300 d'au moins 10 cm, de préférence comprise entre 20 cm et 100 cm, de préférence comprise entre 30 cm et 70 cm, et de préférence encore comprise entre 30 cm et 60 cm.

Spécifiquement lorsque la mise en dépression de la poche de récupération 310 est uniquement créée par l'agencement spécifique par rapport à l'unité de traitement 100, la distance verticale séparant l'unité de traitement 100 de l'unité de récupération 300 est de préférence choisie supérieure à 30 cm, par exemple comprise entre 50 cm et 70 cm, de préférence comprise entre 60 cm et 65 cm, et de préférence encore de l'ordre de 65 cm. Une des particularités du système de traitement proposé réside dans l'unité de traitement 100, amovible par rapport à l'unité de support 10, de manière à pouvoir être remplacer facilement et rapidement, pour chaque nouveau patient, et ayant une configuration permettant de faire un traitement rapide du liquide hémorragique prélevé sans avoir les inconvénients des systèmes existant dans l'art antérieur, notamment ceux basés sur la centrifugation.

Il est à noter que tous les éléments du système de traitement qui sont destinés à être en contact avec le liquide hémorragique à traiter, que l'on qualifie de consommables, sont amovibles par rapport à l'unité de support 10, et peuvent donc être remplacés très facilement. Outre l'unité de traitement 100, cela concerne notamment l'unité de collecte 200, l'unité de récupération 300, et l'unité de transfusion 400.

L'unité de traitement 100 proposée est prévue pour permettre un traitement efficace du liquide hémorragique d'un patient et pour être utilisée plusieurs fois successivement pour un même patient, afin de pouvoir réaliser plusieurs cycles de traitement et ainsi traiter une quantité de liquide hémorragique plus importante.

Comme expliqué plus haut, il est préférable d'effectuer une filtration tangentielle, c'est-à-dire une filtration où le liquide hémorragique à filtrer circule parallèlement à une membrane de filtration et est filtré au contact de cette membrane de filtration.

A cette fin, l'unité de traitement 100 proposée comprend donc un dispositif de filtration 110 pour filtration tangentielle ayant une membrane de filtration 113 agencée dans un boitier 114 de manière à séparer une chambre d'admission 111 d'une chambre d'évacuation 112, la chambre d'admission 111 et la chambre d'évacuation 112 ayant chacune une entrée (111a ;112a) et une sortie (111b; 112b) de fluides. Le liquide hémorragique à traiter circule dans la chambre d'admission 111 depuis l'entrée 111a vers la sortie 111b et subit une filtration tangentielle à travers la membrane de filtration 113 afin de retirer du liquide hémorragique un filtrat comprenant des composés non désirés pour l'autotransfusion. Le filtrat traverse la membrane de filtration 113 jusque dans la chambre d'évacuation 112.

Préférentiellement, le dispositif de filtration 110 pour filtration tangentielle comprend une membrane de filtration 113 à fibres creuses agencée dans le boitier 114, lesdites fibres creuses formant la membrane de filtration s'étendant longitudinalement dans le boitier 114. Le reste de la description est faite essentiellement en référence à une unité de traitement ayant un dispositif de filtration 110 avec une membrane de filtration 113 à fibres creuses mais les enseignements correspondants pourraient s'appliquer à tous types de dispositifs de filtration tangentielle, notamment vis-à-vis des problématiques de colmatage suite aux filtrations successives.

De manière préférée, la membrane de filtration 113 à fibres creuses du dispositif de filtration 110 comprend des fibres formées dans un matériau ayant des propriétés favorisant son hydrophilie. Le fait que la membrane de filtration 113 ait une hydrophilie augmentée permet notamment de réduire le phénomène de colmatage de la membrane qui se fait au fur et à mesure de la filtration. Réduire l'encrassement de la membrane de filtration 113 permet de maintenir une efficacité accrue du dispositif de filtration 110.

Ainsi, de préférence les fibres creuses de la membrane de filtration 113 sont formées à partir d'un mélange de polyester sulfone (PES) et de polyvinyle pyrrolidone (PVP). Par exemple, il est prévu une membrane en PES qui a été mélangée avec du PVP avant extrusion de la fibre. Le matériau de base des fibres creuses pourrait aussi être choisi parmi d'autres matériaux biocompatibles et communément utilisés comme membrane de filtration sanguine, comme par exemple, outre le PES, le polyméthacrylate de méthyle (PMMA), de co ou ter polymère à base d'acrylonitrile.

La membrane de filtration 113 à fibres creuses du dispositif de filtration 110 a par ailleurs de préférence une porosité globale comprise entre 0,1 µm et 1 µm. Une telle taille de pores permet de laisser passer les protéines et autres molécules médicamenteuses qui sont impropres à être transfusées, tout en permettant de conserver les composés d'intérêt du liquide hémorragique, à savoir les globules rouges, les globules blancs et les plaquettes.

Dans le dispositif de filtration 110 proposé, la membrane de filtration 113 a par exemple une surface de filtration globale supérieure à 0,04 m², par exemple comprise entre 0,1 m² et 3 m², et de préférence comprise entre 0,2 m² et 0,6 m². Plus précisément, la surface de filtration est choisie de sorte à être à la fois suffisante pour permettre une filtration rapide du liquide hémorragique, typiquement en 5 minutes ou moins, mais pas trop importante pour éviter une trop grande adhésion protéique et donc une perte en plaquette associée. De préférence, on choisit le dispositif de filtration permettant une filtration en moins de 5 min pour le traitement d'un volume de 500 ml de liquide hémorragique.

Le dispositif de filtration peut selon un premier exemple comprendre une membrane de filtration à fibres creuses agencées longitudinalement dans un boitier cylindrique, la membrane de filtration ayant une porosité moyenne de 0,6 µm, une surface de filtration de 0,2 m², lesdites fibres creuses étant formées à partir d'un mélange de polyester sulfone (PES) et de polyvinyle pyrrolidone (PVP), ayant un diamètre interne de 300 µm, un diamètre externe de 470 µm, et une paroi de 85 µm d'épaisseur.

Selon un deuxième exemple, le dispositif de filtration a les mêmes caractéristiques que selon le premier exemple mais avec une surface de filtration de 0,6 m².

Selon un troisième exemple, le dispositif de filtration a les mêmes caractéristiques que selon le premier exemple mais avec une surface de filtration de 0,4 m².

L'unité de traitement 100 comprend en outre une poche de traitement 140 qui est reliée fluidiquement au dispositif de filtration 110 par une ligne de recirculation 150.

Plus précisément, la poche de traitement 140 a une entrée 140a et une sortie 140b reliées fluidiquement par une ligne de recirculation 150 à la sortie 111b et à l'entrée 111a de la chambre d'admission 111 du dispositif de filtration 110, respectivement.

Cet agencement permet notamment une circulation du liquide hémorragique dans la ligne de recirculation 150 dans un sens allant de la sortie 140b de la poche de traitement 140 vers l'entrée 140a de la poche de traitement 140 à travers la chambre d'admission 111 du dispositif de filtration 110.

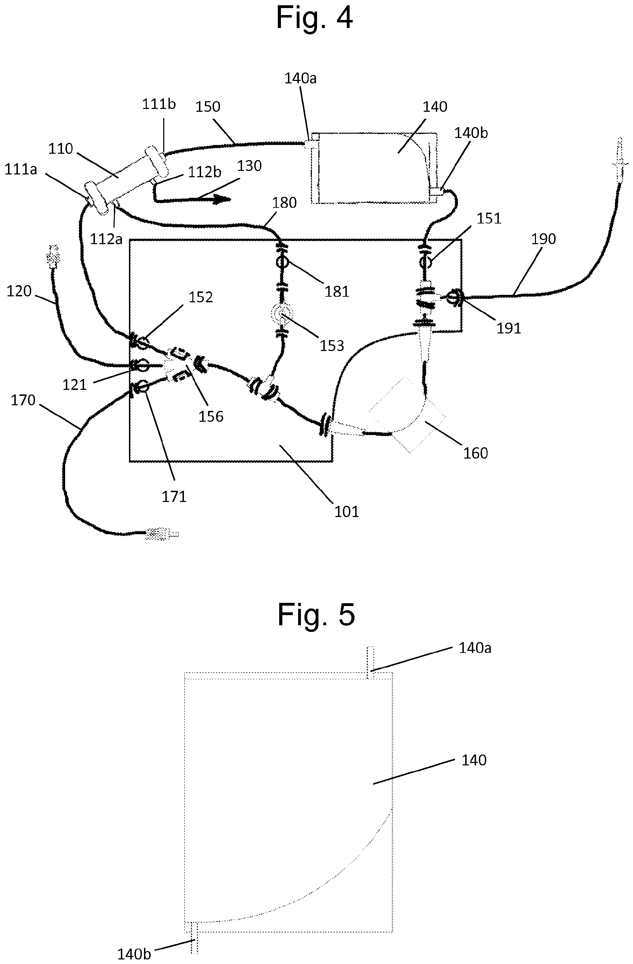

La poche de traitement 140 a une forme qui est également prévue pour favoriser le flux interne depuis l'entrée 140a vers la sortie 140b et favoriser le mélange du liquide sanglant traité au dépend d'un effet de sédimentation et de circulation privilégié en fond de poche. Par exemple, comme illustré à la figure 5,

La poche de traitement 140 a une forme sensiblement parallélépipédique avec l'entrée 140a et la sortie 140b de part et d'autre de la poche de traitement selon une diagonale. De préférence encore, la proche de traitement présente en outre une cavité interne ayant une forme rétrécie du côté de la sortie 140b, de sorte à faire converger le liquide contenu dans la poche de traitement 140 vers la sortie 140b.

Cette poche de traitement 140 a un rôle actif dans le cycle de traitement du liquide hémorragique. Tout d'abord, comme on le verra plus loin, elle permet une recirculation du liquide hémorragique pendant un cycle de traitement, c'est-à-dire plusieurs circulations successives dans le dispositif de filtration 110, sans variation du débit de circulation du liquide hémorragique pendant ledit cycle de traitement. Elle joue en effet le rôle de zone tampon qui permet d'absorber les variations éventuelles du flux. La poche de traitement 140 pourra également être utilisée comme zone de mélange du liquide hémorragique à traiter avec un liquide de dilution afin de favoriser la filtration et l'élimination d'éléments solubles tels que les protéines et les substances médicamenteuses au travers du dispositif de filtration 110.

La poche de traitement 140 peut être équipée, sans que cela ne soit obligatoire, d'un dispositif séparateur 141 pouvant être actionné pour séparer la poche de traitement 140 en une première chambre de traitement 142 du côté de l'entrée 140a de la poche de traitement 140 et une deuxième chambre de traitement 143 du côté de la sortie 140b de la poche de traitement 140. Un tel dispositif séparateur 141 peut par exemple prendre la forme d'un clamp électromécanique pouvant être actionné pour former lesdites première et deuxième chambres de traitement en fonction du déroulement du cycle de traitement.

Les moyens d'entrainement de liquide de l'unité de support 10 sont prévus pour effectuer principalement une circulation du liquide hémorragique dans le sens cité précédemment - appelé sens de traitement - allant de la sortie 140b de la poche de traitement 140 vers l'entrée 140a de la poche de traitement 140 à travers la chambre d'admission 111 du dispositif de filtration 110. Ils peuvent éventuellement aussi permettre une circulation en sens inverse pour certaines phases spécifiques du cycle de traitement comme on le verra plus loin.

Selon l'exemple de réalisation de la figure 2, il est prévu une pompe péristaltique 160 dans la ligne de recirculation positionnée dans la ligne de recirculation 150 entre la sortie 140b de la poche de traitement 140 et l'entrée 111a de la chambre d'admission 111 du dispositif de filtration 110. Elle permet de faire circuler le liquide hémorragique dans les deux sens de circulation précisés ci-dessus.

L'unité de traitement 100 comprend par ailleurs divers conduits permettant la circulation de liquide au sein même de l'unité de traitement 100 avec la ligne de circulation 150 mentionnée précédemment, mais également vers/depuis les autres unités du système de traitement.

Ainsi, l'unité de traitement 100 comprend une ligne d'admission 120 reliée fluidiquement à la ligne de recirculation 150 entre la sortie 140b de la poche de traitement 140 et l'entrée 111a de la chambre d'admission 111 du dispositif de filtration 110 permettant d'alimenter l'unité de traitement 110 avec le liquide hémorragique prélevé en vue d'une filtration à travers la membrane de filtration 113 à fibres creuses du dispositif de filtration 110 afin de retirer du liquide hémorragique un rétentat comprenant des composés non désirés pour l'autotransfusion. Cette ligne d'admission 120 est par ailleurs destinée à être reliée, de manière amovible, à l'unité de collecte 200 présentée plus haut.

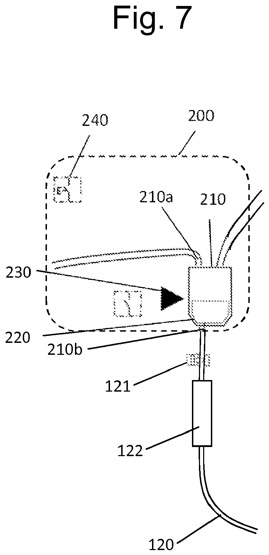

Comme illustré à la figure 7, il peut être prévu d'interposer dans cette ligne d'admission 120, du côté destiné à être relié à l'unité de collecte 200, un dispositif de préfiltration supplémentaire 122 permettant de réaliser une filtration additionnelle avant la filtration effective par le dispositif de filtration 110 de l'unité de traitement 100. Un tel dispositif de préfiltration supplémentaire 122 a pour objet de retenir la masse de substance coagulée, appelée coagulum, qui est susceptible de se former à la sortie du réceptacle de collecte 210 malgré l'éventuel dispositif de préfiltration 220.

Le dispositif de préfiltration supplémentaire 122 fonctionne comme un filtre dynamique, c'est-à-dire qu'il doit pouvoir fonctionner avec les débits imposés par l'unité de traitement, sans venir détériorer les performances de temps de traitement. Le dispositif de préfiltration supplémentaire 122 a de préférence un niveau de filtration plus élevé que le niveau de filtration de l'éventuel dispositif de préfiltration 220 du réceptacle de collecte 210.

Le dispositif de préfiltration supplémentaire 122 peut par exemple avoir un niveau de filtration compris entre 40 µm et 200 µm, de préférence compris entre 100 µm et 170 µm, de préférence encore de l'ordre de 150 µm. Le volume de rétention de coagulum peut être compris entre 5 ml et 100 ml, et de préférence compris entre 20 ml et 50 ml.

De préférence, ce dispositif de préfiltration supplémentaire 122 fait partie intégrante de l'unité de traitement 100. Il peut toutefois être également envisagé que ce dispositif de préfiltration supplémentaire soit intégré à l'unité de collecte 200, au niveau de la sortie du réceptacle de collecte 210.

De préférence, ce dispositif de préfiltration supplémentaire 122 est monté de manière amovible dans le système de traitement, ce qui permet par exemple de pouvoir le retirer et le nettoyer en cas de colmatage.

L'unité de traitement 100 comprend en outre une ligne de transfusion 170 également reliée fluidiquement à la ligne de recirculation 150 entre la sortie 140b de la poche de traitement 140 et l'entrée 111a de la chambre d'admission 111 du dispositif de filtration 110, et permettant de récupérer le liquide hémorragique traité contenu dans ladite poche de traitement 140. Cette ligne de transfusion 170 est par ailleurs destinée à être reliée, de manière amovible, à l'unité de transfusion 400 présentée plus haut.

De manière préférée, la ligne d'admission 120 et la ligne de transfusion 170 sont piquées à la même position sur la ligne de recirculation 150 comme cela est illustré à la figure 2, avec par exemple avec un connecteur fluidique multivoies 156. Il peut ainsi être utilisé un connecteur 156 à 3 voies comme illustré aux figures 2, 3, et 4, avec une voie d'entrée reliée fluidiquement à la ligne de recirculation 150 en direction de la sortie 140b de la poche de traitement 140, une première voie de sortie reliée fluidiquement à la ligne de recirculation 150 en direction de l'entrée 111a de la chambre d'admission 111 du dispositif de filtration 110, une deuxième sortie reliée fluidiquement à la ligne d'admission 120, et une troisième sortie reliée fluidiquement à la ligne de transfusion 170.

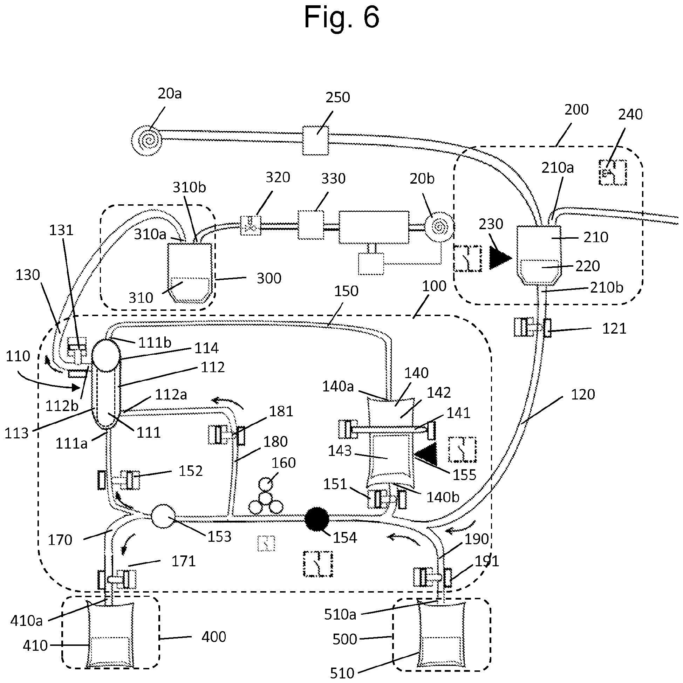

Comme on le verra plus loin, et tel qu'illustré à la figure 6, il pourrait toutefois être prévu que la ligne d'admission soit piquée en amont de la pompe péristaltique 160, au niveau de la sortie 140b de la poche de traitement 140. Dans ce cas, la ligne de transfusion est reliée à la ligne de recirculation par un connecteur fluidique à 2 voies standard. Dans ce cas, il n'est pas nécessaire que la pompe péristaltique 160 soit adaptée pour faire circuler le liquide hémorragique dans les deux sens de circulation et il est suffisant d'avoir une pompe permettant de faire circuler le liquide hémorragique selon un seul sens, à savoir le sens de traitement.

L'unité de traitement 100 comprend par ailleurs une ligne d'évacuation 130 reliée fluidiquement à la sortie 112b de la chambre d'évacuation 112 du dispositif de filtration 110 de sorte à évacuer le filtrat ayant traversé la membrane de filtration 113 à fibres creuses depuis la chambre d'admission 111. Cette ligne d'évacuation 130 est par ailleurs destinée à être reliée, de manière amovible, à l'unité d'évacuation 130 présentée plus haut.

Lorsque la poche de récupération 310 est prévue pour être mise en dépression, par un système de vide par exemple, cela permet d'accélérer la filtration à travers la membrane de filtration 113 du dispositif de filtration 100 puisque ladite dépression se répercute à travers la ligne d'évacuation 130 jusque dans la chambre d'évacuation 112.

L'unité de traitement 100 peut par ailleurs comprendre une ligne de nettoyage 180 reliée fluidiquement à l'entrée 112a de la chambre d'évacuation 112 du dispositif de filtration 110 pour amener un liquide de nettoyage dans ladite chambre d'évacuation 112. Il est à noter que cette ligne de nettoyage 180 n'est pas obligatoire dans tous les exemples de réalisation présentés.

L'objectif de cette ligne de nettoyage 180 est de permettre d'amener un liquide dans la chambre d'évacuation 112 et de créer un contre-flux transmembranaire, c'est-à-dire un flux allant dans un sens inverse du sens habituel du flux à travers la membrane de filtration 113 à fibres creuses du dispositif de filtration 110 pendant la filtration. Ce contre-flux à travers la membrane de filtration 113 est très utile pour décoller tout ou partie des éléments retenus sur les fibres creuses de la membrane de filtration 113, et ainsi régénérer les capacités de filtration de la membrane de filtration 113 notamment en termes d'efficacité et rapidité de filtration. Le contre-flux proposé est également avantageux en ce qu'il permet le nettoyage de la membrane de filtration de façon simple et rapide, pendant un cycle de traitement ou entre deux cycles de traitement.

Il peut être prévu un organe de régulation de flux 181 agencé pour une régulation de flux dans la ligne de nettoyage 180 et un autre organe de régulation de flux 131 agencé pour une régulation de flux dans la ligne de d'évacuation 130 de manière à pouvoir contrôler la pression de liquide de nettoyage dans la chambre d'évacuation 112.

Le contre-flux peut ainsi être créé en obstruant la ligne d'évacuation 130 au niveau de l'organe de régulation de flux 131 et en injectant le liquide de nettoyage dans la chambre d'évacuation 112 depuis la ligne de nettoyage 180, la pression créée dans la chambre d'évacuation 112 par l'injection du liquide de nettoyage créant un contre-flux transmembranaire requis.

Le liquide de nettoyage peut être injecté directement dans la ligne de nettoyage 180 lorsque cela est requis pour créer le contre-flux, une source externe contenant le liquide de nettoyage étant alors prévu.

Que l'unité de traitement 100 comprenne une ligne de nettoyage 180 ou non, il peut en outre être prévu une ligne de dilution 190 permettant d'amener un liquide de dilution dans l'unité de traitement 100. La ligne de dilution 190 est alors de préférence reliée fluidiquement à la ligne de recirculation 150 à une position entre la sortie 140b de la poche de traitement 140 et l'entrée 111a de la chambre d'admission 111 du dispositif de filtration 110. De préférence, la ligne de dilution 190 est piquée en amont de la pompe péristaltique 160.

Cette ligne de dilution 190 est par ailleurs destinée à être reliée, de manière amovible, à une unité de dilution 500 comprenant un réceptacle de dilution 510 ayant un orifice d'entrée/sortie 510a destiné à être couplé à la ligne de dilution. Le réceptacle de dilution 510 contient le liquide de dilution qui est destiné à être injecté dans l'unité de traitement 100. Ce liquide de dilution peut être une composition cristalloïde, de préférence une solution isotonique compatible avec les hématies, sans glucides ni protéines, comprenant par exemple du chlorure de sodium, du lactate de sodium, et/ou du chlorure de potassium dilué dans de l'eau pour réaliser une préparation injectable.

Un organe de régulation de flux 191 est de préférence prévu pour permettre une régulation de flux dans la ligne de dilution 190.

De manière préférée, la ligne de nettoyage 180 est reliée fluidiquement à la ligne de recirculation 150 à une position entre la sortie 140b de la poche de traitement 140 et l'entrée 111a de la chambre d'admission 111 du dispositif de filtration 110. Lorsque l'unité de traitement 100 comprend une ligne de dilution 190 alors le liquide de dilution peut être utilisé pour alimenter la ligne de nettoyage 180 et ainsi être utilisé en tant que liquide de nettoyage.

De préférence, un autre organe de régulation de flux 151 est prévu dans l'unité de traitement 100 et agencé pour une régulation de flux dans la ligne de recirculation 150 au niveau de la sortie 140b de la poche de traitement 140.

Encore un autre organe de régulation de flux 152 est de préférence prévu de manière à pouvoir effectuer une régulation de flux dans la ligne de recirculation 150 au niveau de l'entrée 111a de la chambre d'admission 111 du dispositif de filtration 110.

Il peut également être prévu un organe de régulation de flux 171 agencé pour une régulation de flux dans la ligne de transfusion 170, permettant ainsi de contrôler le flux à transmettre à l'unité de transfusion 400.

L'exemple de réalisation présenté aux figures 2 à 4 et 6 est un système de traitement dont l'unité de traitement 100 nécessite une unique pompe péristaltique 160 pour la circulation des liquides dans le circuit, cette pompe péristaltique 160 étant agencée de manière à faire circuler le liquide hémorragique dans la ligne de recirculation 150 dans un sens - dit sens de traitement - allant de la sortie 140b de la poche de traitement 140 vers l'entrée 140a de la poche de traitement 140 à travers la chambre d'admission 111 du dispositif de filtration 110.

Préférentiellement cette pompe péristaltique 160 permet de faire circuler le flux également dans un sens inverse au sens de traitement, c'est-à-dire allant de l'entrée 111a de la chambre d'admission 111 du dispositif de filtration 110 vers la sortie 140b de la poche de traitement 140. Cela est particulièrement avantageux dans l'exemple de réalisation de la figure 2 où la ligne d'admission 120 est piquée en aval de la pompe péristaltique 160, c'est-à-dire au niveau de l'entrée 111a de la chambre d'admission 111 du dispositif de filtration 110.De préférence, la pompe péristaltique 160 est positionnée dans la ligne de recirculation 150 entre la sortie 140b de la poche de traitement 140 et l'entrée 111a de la chambre d'admission 111 du dispositif de filtration 110 entre la position où la ligne de dilution 190 est reliée fluidiquement à la ligne de recirculation 150 et la position où la ligne de nettoyage 180 est reliée fluidiquement à la ligne de recirculation 150.

En fonction du fonctionnement envisagé de l'unité de traitement 100, la ligne d'admission 120 est piquée sur la ligne de recirculation 150 en aval de la pompe péristaltique 160 comme illustré à la figure 2, c'est-à-dire du côté de l'entrée 112a de la chambre d'admission 112 du dispositif de filtration, ou est piquée sur la ligne de recirculation 150 en amont de la pompe péristaltique 160, c'est-à-dire du côté de la sortie 140b de la poche de traitement 140, comme illustré à la figure 6.

Dans le cas, où la ligne d'admission 120 est piquée sur la ligne de recirculation 150 en aval de la pompe péristaltique 160 comme illustré à la figure 2, le liquide hémorragique sera, lors du lancement d'un cycle de traitement, transmis de préférence du réceptacle de collecte 210 vers la poche de traitement 140.

Dans le cas, où la ligne d'admission 120 est piquée sur la ligne de recirculation 150 en amont de la pompe péristaltique 160, le liquide hémorragique sera, lors du lancement d'un cycle de traitement, transmis de préférence du réceptacle de collecte 210 directement à travers le dispositif de filtration 110. Cela présente l'avantage de ne pas nécessiter une pompe péristaltique 160 devant fonctionner dans les deux sens de circulation du liquide.

Comme il a été précisé plus haut, le système de traitement comprend de préférence des capteurs permettant de suivre l'évolution du traitement au cours d'un cycle de traitement spécifique.

Il peut à cet égard être prévu un capteur de pression 153 au sein de la ligne de circulation 150, agencé notamment pour détecter des surpressions en aval de la pompe péristaltique 160 dans le sens de traitement.

Un capteur d'hématocrite 154 peut en outre être prévu pour mesurer le taux d'hématocrite du liquide circulant dans l'unité de traitement 100. Un tel capteur d'hématocrite 154 peut par exemple être un capteur optique formé d'un assemblage de diodes émettrices dans l'infra-rouge et de récepteurs.

Il peut aussi être prévu un système de pesée 155, tel qu'un capteur d'effort formant peson, agencé pour mesurer la quantité de liquide présent dans la poche de traitement 140. Ce système de pesée 155 va permettre de donner des informations pour commander le cycle de traitement, permettant par exemple d'enclencher un transfert du liquide hémorragique traité vers l'unité de transfusion 400 lorsque les paramètres cibles, notamment en termes de taux d'hématocrite, sont atteints.

L'unité de traitement de 100 étant un consommable, destiné à être remplacer pour chaque nouveau patient notamment, il est préférable que sa manutention soit simple et aisée. Comme cette unité de traitement 100 comprend un certain nombre de composants différents et des tubulures formant les lignes de circulation de liquide, il peut être prévu un gabarit 101 qui permet notamment une fixation de la ligne d'admission 120, la ligne d'évacuation 130, la ligne de recirculation 150, la ligne de transfusion 170, ainsi que la ligne de nettoyage 180 et/ou la ligne de dilution 190 le cas échéant. Le dispositif de filtration 110 ainsi que la poche de traitement 140 sont parties intégrantes de l'unité de traitement 100 et sont donc préalablement connectées aux tubulures correspondantes et au gabarit 101. L'unité de traitement 100 ainsi proposée peut donc être proposée comme un consommable sous forme de kit, à remplacer selon les besoins.

Le gabarit 101 est également prévu pour permettre une mise en place aisée sur l'unité de support 10 et a à cet égard une forme en détrompeur permettant de coupler l'unité de traitement 100 à l'unité de support 10 selon un unique positionnement.

Par ailleurs, le dispositif de filtration 110 de l'unité de traitement 100 est destiné à être couplé à l'unité de support 10 de sorte que les fibres creuses de la membrane de filtration 113 s'étendent selon une direction non comprise dans le plan de support horizontal défini par l'unité de support 10, c'est-à-dire que le dispositif de filtration 110 est incliné par rapport au plan de support horizontal. Il est à noter que ce plan de support horizontal correspond au plan transverse de l'unité de support 10, qui est parallèle au plan horizontal lorsque l'unité de support 10 est posée au sol.

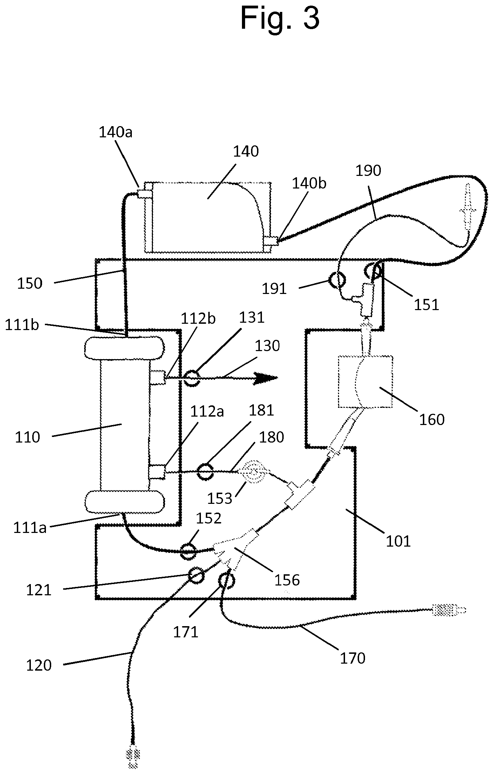

L'exemple de la figure 3 illustre une unité de traitement 100 ayant un gabarit 101 prévu pour une mise en place verticale du gabarit 101, c'est-à-dire que le gabarit 101 est fixé dans un plan différent du plan de support horizontal de l'unité de support 10, par exemple un plan incliné, et de préférence un plan vertical (c'est-à-dire perpendiculaire au plan de support horizontal). Selon ce mode de réalisation, le dispositif de filtration 110 peut également être fixé au gabarit 101 puisque la mise en place du gabarit 101 entrainera nécessairement un agencement incliné du dispositif de filtration 110.

L'exemple de la figure 4 illustre une unité de traitement 100 ayant un gabarit 101 prévu pour une mise en place horizontale du gabarit 101, c'est-à-dire que le gabarit 101 est fixé dans un plan parallèle au plan de support horizontal de l'unité de support 10. Selon ce mode de réalisation, le dispositif de filtration 110 n'est pas fixé au gabarit 101 et devra être fixé à l'unité de support 10 de manière indépendante pour être agencé incliné par rapport au plan de support horizontal.

Comme illustré aux figures 3 et 4, les organes de régulation de flux qui ont été décrits précédemment peuvent prendre la forme d'orifices agencés en vis-à-vis des tubulures formant les lignes de circulation de liquide où le flux doit être contrôlé. Il est alors prévu des valves de régulation, par exemple des électrovannes fonctionnant avec des électroaimants, agencées pour venir contre les tubulures à travers les orifices afin de contrôler la section des tubulures et donc le flux de liquide pouvant les traverser. De manière préférées, ces valves de régulation sont directement montées sur l'unité de support 10 et peuvent donc être utilisées pour différentes unités de traitement 100 successives.

La mise en place de l'unité de traitement 100 incluant un gabarit 101 est très simple. Il suffit en effet de positionner le gabarit 101 dans le logement prévu à cet effet sur le support. Lorsque les organes de régulation de flux sont des orifices destinés à coopérer avec des électrovannes de l'unité de support 101, la mise en vis-à-vis est automatique grâce à la forme en détrompeur du gabarit 101. Une fois le gabarit 101 en place sur l'unité de support 10, il convient de connecter la ligne d'admission 120 à l'unité d'admission 200, la ligne d'évacuation 130 à l'unité d'évacuation 300, la ligne de transfusion 170 à l'unité de transfusion 400, et éventuellement la ligne de dilution 190 à l'unité de dilution 500. Il est à noter que certaines des unités pourraient être préconnectées à leur ligne respective, c'est-à-dire être déjà connectées à l'unité de traitement 100 avant mise en place sur l'unité de support 10. Par exemple l'unité de transfusion 400 est de préférence préconnectée à la ligne de transfusion 170, et l'unité d'évacuation 300 peut être aussi préconnectée à la ligne d'évacuation 130. Lorsque le dispositif de filtration 110 et/ou la poche de traitement 140 ne sont pas fixés au gabarit 101, il convient de les fixer sur l'unité de support 10.

Le débit de traitement est choisi suffisamment élevé pour appliquer une force de cisaillement importante venant limiter l'adhésion protéique et plaquettaire au niveau du dispositif de filtration, sans toutefois être trop importante ce qui créerait une hémolyse non désirée. Les débits choisis sont très supérieurs, généralement de 5 à 10 fois supérieurs, aux débits généralement préconisés pour un dispositif de filtration utilisant une membrane de filtration à fibres creuses. Il a été constaté en effet que les résultats de filtration étaient très positifs en termes de concentration cellulaire, et les débits de circulation élevés ne sont pas gênants puisque le système de traitement n'est pas relié directement au patient, mais uniquement indirectement via l'unité de collecte 200 d'une part et l'unité de transfusion 400 d'autre part lorsque ces dernières sont connectées au patient. En pratique, comme indiqué plus haut, l'unité de traitement 100 fonctionne de préférence avec un débit compris entre 10 ml/min et 4000 ml/min, et de préférence entre 100 ml/min à 2100 ml/min, par exemple à 1400 ml/min ou 700 ml/min.

L'application d'un vide contrôlé entre 0 à -100 kPa au niveau de l'unité de récupération 300 à travers la ligne d'évacuation 130 permet d'améliorer la vitesse de filtration et de la maintenir constante. De plus, lors d'une inversion des flux transmembranaire (notamment lors d'un nettoyage à contre flux) un désamorçage de la filtration peut être observé si une dépression n'est pas appliquée en continue, rendant ainsi impossible la poursuite du traitement du sang.

Outre les avantages mentionnés plus haut en termes de nettoyage pour allonger la durée de vie et le rendement filtrant du dispositif de filtration 110, le passage du liquide de dilution pour rincer la membrane de filtration 113 à fibres creuses entre deux cycles de traitement participe aussi au recouvrement plaquettaire.

Le système de traitement proposé pour le traitement de liquide hémorragique en vue d'une autotransfusion est simple d'utilisation et permet un traitement rapide du liquide hémorragique prélevé chez le patient avec des performances qualitatives supérieures aux dispositifs existants. Tout ou partie des performances indiquées ci-dessous peuvent en effet être atteintes avec le dispositif de traitement proposé :
- Rendement moyen en plaquettes supérieur ou égal à 40%, voire supérieur ou égal à 50%, voire supérieur ou égal à 60%, et même supérieur ou égal à 70% ;
- Rendement moyen en globules rouges (GR) supérieur ou égal à 80%, voire supérieure ou égal à 90%, et même supérieur ou égal à 95%, voire même de l'ordre de 99% ;
- Rendement moyen en globules blancs (GB) supérieur ou égal à 80%, voire supérieure ou égal à 90%, et même supérieur ou égal à 95%, voire même de l'ordre de 97% ;
- Hémolyse réduite, voire hémolyse nulle ou proche de zéro. L'hémolyse cible pourra par exemple être inférieure à 1%, de préférence inférieure à 0,8% ;
- Elimination de l'hémoglobine libre supérieure ou égale à 95%, voire supérieure ou égale à 98 % ;
- Concentration résiduelle d'héparine dans la poche de transfusion après traitement inférieure ou égale à 0.5 UI/ml.
- Temps de traitement d'un volume de 500 ml de liquide hémorragique pour le rendre propre à une transfusion qui est inférieur ou égal à 10 min, de préférence inférieur ou égal à 8 min, de préférence inférieur ou égal à 6 min, et de manière optimum de l'ordre de ou inférieur ou égal à 5 min.

Il est à noter que le niveau des performances qualitatives indiquées ci-dessus est à moduler en fonction des conditions opératoires.

Par exemple, si le traitement doit être fait très rapidement, il est possible que les performances soient un peu diminuées.

De la même manière, si l'on décide de concentrer plusieurs fois le liquide hémorragique traité, par exemple lors d'une triple concentration, afin d'être certain de supprimer les composés non désirés pour l'autotransfusion, tels que les produits indésirables à la transfusion, de type héparine, il est possible que certaines des performances soient diminuées, sans que cela ne vienne toutefois réduire la performance globale du système de traitement proposé par rapport aux systèmes existants.

### Fonctionnement du système de traitement d'un liquide hémorragique en vue d'une autotransfusion

Il est présenté ci-dessous un exemple de fonctionnement du système de traitement proposé, selon un mode standard, et n'est en aucun cas limitatif. Le système de traitement proposé pourrait en effet être utilisé en mode standard selon des phases spécifiques différentes, adaptées en fonction du besoin de l'intervention chirurgicale ou du besoin en transfusion. Le fonctionnement du système de traitement proposé peut également être adapté à des situations opérationnelles particulières, par exemple en urgence lorsqu'une transfusion est nécessaire même si le traitement n'est pas totalement terminé, ou lorsque le volume de liquide hémorragique à traiter n'est pas optimal pour le cycle de traitement standard.

L'exemple de fonctionnement ci-dessous est présenté en référence au premier agencement de l'unité de traitement 100 tel qu'illustré aux figures 2 à 4. Selon cet agencement, il est possible de diluer le liquide hémorragique avant de lancer la concentration par passage à travers le dispositif de filtration. Le volume de liquide de rinçage utilisé pour la dilution peut être de 200 ml pour un bolus de liquide hémorragique de 500 ml, mais ce volume de liquide de rinçage pourrait être beaucoup plus élevé, par exemple de l'ordre de 18 litres (selon la concentration initiale en anticoagulant dans le liquide hémorragique). Ce volume de dilution peut être ajouté en début de traitement ou par bolus de quelques ml à quelques centaines de ml au cours du traitement, après une première concentration ou non du volume de liquide hémorragique.

Dans un exemple de fonctionnement selon le deuxième agencement de l'unité de traitement 100 tel qu'illustré à la figure 6, le liquide hémorragique entrant dans l'unité de traitement est concentré, en passant par le dispositif de filtration, avant l'ajout du liquide de rinçage. En effet, le liquide hémorragique traverse directement le dispositif de filtration 113 lors de son transfert de l'unité de collecte 200 vers la poche de traitement 140. L'avantage d'une filtration avant ajout de liquide de dilution est une élimination directe des éléments solubles pendant le transfert vers la poche de traitement. Dans ce cas, le fait de pouvoir décolmater le dispositif de filtration est particulièrement avantageux puisqu'il aura tendance à s'encrasser plus rapidement.

### A. Phase de préparation du système de traitement

Nous allons dans un premier temps décrire la phase de préparation du système de traitement proposé, comprenant une phase d'installation des composants du système de traitement puis une phase d'initialisation et de test de ce système de traitement.

Tous les clamps formés par les électrovannes pour la régulation des flux de liquide sont en position ouverte.

On installe l'unité de collecte 200, l'unité de récupération 300, l'unité de transfusion 400 ainsi que l'unité de dilution 500 sur l'unité de support 10.

On installe ensuite l'unité de traitement 100 sur l'unité de support 10. Le clamp de la valve de régulation 121 de la ligne d'admission 120 et celui de la valve de régulation 191 de la ligne de dilution 190 sont fermés.

On connecte enfin les différentes unités grâce aux tubulures des lignes de circulation de liquide correspondantes, et connecteurs associés, ces connecteurs étant par exemple de type « Luer Lock ».

L'unité de collecte 200 ainsi que l'unité de récupération 300 sont connectées aux prises murales de vide (20a ; 20b). Une source d'une solution cristalloïde héparinée peut par ailleurs être connectée à l'entrée 210a du réceptacle de collecte 210.

Une fois que tous les éléments ont été connectés à l'unité de support 10, le système de traitement peut être mise en marche (alimentation électrique) et une phase d'initialisation et de test du système de traitement se lance, pilotée par l'unité centrale de l'unité de support 10.

Dans cette phase de test, il est vérifié que toutes les unités sont bien connectées à l'unité de support 10 et entres elle. Il peut par exemple être utilisé des contacts optiques placés au niveau de l'unité de support 10 notamment pour que l'unité centrale obtienne ce type d'information.

En initialisation, une commande de l'unité centrale contrôle les clamps des différentes lignes pour que les valves de régulation soient toutes en position fermée.

Les sources de vides sont maintenant alimentées et le système de traitement est opérationnel pour lancer un cycle de traitement.

### B. Phase préparatoire du circuit de l'unité de traitement avant premier cycle de traitement

Avant de lancer un cycle de traitement à proprement parlé, il peut être effectué, de manière préférée mais optionnelle, une phase préparatoire du cycle de traitement qui permet d'améliorer l'efficacité du cycle de traitement à proprement parlé.

Cette phase préparatoire peut être faite en amont de l'intervention chirurgicale, mais il est préféré de la faire pendant l'intervention chirurgicale, dès que des saignements sont constaté et qu'une autotransfusion est envisagée.

On effectue tout d'abord un amorçage de l'unité de collecte 200 en remplissant le réceptacle de collecte 210 par aspiration de la solution cristalloïde héparinée jusqu'à obtenir un certain volume dans ledit réceptacle de collecte 210. Cette aspiration peut par exemple être réalisée par application d'un vide dans le réceptacle de collecte 210 de l'ordre de 300 mbar. On remplit le réceptacle de collecte 210 avec par exemple 200 ml de la solution cristalloïde héparinée. Cet amorçage de l'unité de collecte avec la solution cristalloïde héparinée permet d'humidifier l'unité de collecte 200 ce qui va faciliter la préfiltration en son sein, en termes de vitesse de filtration notamment. En outre cela permet d'hépariner les matériaux et donc limiter le phénomène de coagulation.

On effectue ensuite un amorçage de la ligne de recirculation de l'unité de traitement 100 dans laquelle le traitement à proprement parlé est effectué. Cet amorçage peut débuter notamment lorsque le saignement est actif. Pour ce faire, on active la mise en dépression de l'unité de récupération 300, par exemple un vide est appliqué à travers le dispositif de filtration 100 en activant la vanne 320, tandis que la valve de régulation 121 de la ligne d'admission 120 et de la valve de régulation 191 de la ligne de dilution 190 étant fermés et tous les autres clamps ouverts. On ferme ensuite les clamps de la valve de régulation 181 de la ligne de nettoyage 180, de la valve de régulation 151 de la ligne de recirculation 150, de la valve de régulation 171 de la ligne de transfusion 170, et on ouvre le clamp de la valve de régulation 191 de la ligne de dilution 190. La pompe péristaltique 160 est mise en marche et la ligne de recirculation 150 se remplit avec le liquide de dilution de l'unité de dilution 500. L'air présent dans le circuit est évacué par la ligne d'évacuation 130 et le liquide de dilution remplit progressivement les différentes chambres vides des éléments de l'unité de traitement 100, en particulier du dispositif de filtration 110 et de la poche de traitement 140.

Après une temporisation de remplissage de la ligne de recirculation, on effectue le cas échéant un amorçage de la ligne de nettoyage 180. A cet égard, tandis que la dépression de l'unité de récupération 300 est toujours active, avec par exemple le vide appliqué à travers l'unité de récupération 300, on ouvre le clamp de la valve de régulation 181 de la ligne de nettoyage 180 puis on ferme les clamps de la valve de régulation 152 de la ligne de recirculation 150 et de la valve de régulation 151 de la ligne de recirculation 150. Le liquide de dilution circule alors à travers la ligne de nettoyage 180 jusque dans la chambre d'évacuation 112 du dispositif de filtration 110 puis à travers les fibres creuses de la membrane de filtration 113 en contre-flux.

Cet amorçage de la ligne de recirculation et de la ligne de nettoyage 180 de l'unité de traitement 100 remplit progressivement la poche de traitement 140 avec le liquide de dilution.

Préférentiellement, on poursuit ces phases d'amorçage jusqu'à ce qu'un certain volume du liquide de dilution soit présent dans la poche de traitement 140, ce liquide servira à diluer/laver le liquide hémorragique à traiter. De préférence, le clamp de la valve de régulation 131 de la ligne d'évacuation 130 est fermé, et la vanne 320 pour le vide est également fermée, pour remplir plus rapidement la poche de traitement 140 avec le liquide de dilution. Dans la poche de traitement, le remplissage avec le liquide de dilution se poursuit pour atteindre un volume - dit volume de lavage - utile pour le premier cycle de traitement. On utilise par exemple un volume de lavage de 200 ml lorsque l'on souhaite traiter un bolus de liquide hémorragique d'environ 500 ml. Lorsque le peson 155 de la poche de traitement 144 détecte que le poids (=volume) de lavage est atteint, la pompe 160 est arrêtée.

Il pourrait être aussi envisagé de ne pas remplir la poche de traitement 140 et au contraire de la vider via l'unité d'évacuation 300, après la phase d'amorçage. Dans ce cas, la dilution du bolus de liquide hémorragique se fera directement pendant la phase de traitement, lorsque le liquide hémorragique est injecté dans le circuit de l'unité de traitement 100.

L'unité de traitement 100 est ainsi prête pour recevoir un premier bolus de liquide hémorragique à traiter et effectuer le premier cycle de traitement. En attendant que le réceptacle de collecte 210 contienne une quantité suffisante de liquide hémorragique pour le premier cycle de traitement (par exemple environ 500 ml), les clamps de la valve de régulation 181 de la ligne de nettoyage 180 et de la valve de régulation 191 de la ligne de dilution 190 se ferment, de sorte que tous les clamps sont fermés.

C'est le peson 230 du réceptacle de collecte 210 qui déclenchera le lancement du traitement quand la quantité de liquide hémorragique à traiter sera suffisante.

Il est à noter que l'ordre d'ouverture/fermeture des différents clamps est choisi pour permettre que la pompe fonctionne en continue et avoir donc une circulation continue des liquides dans l'unité de traitement 100.

### C. Phase de traitement du liquide hémorragique

Dans le processus global de l'autotransfusion, il y a trois étapes successives indépendantes les unes des autres :
E1. Prélèvement du liquide hémorragique, où une intervention auprès du patient est nécessaire. Le liquide hémorragique est en effet prélevé auprès du patient et transféré dans le réceptacle de collecte 210 de l'unité de collecte 200.
E2. Traitement du liquide hémorragique prélevé, qui se fait sans lien avec le patient. Cette étape de traitement est faite au système de traitement proposé, notamment dans l'unité de traitement 100.
E3. Transfusion du liquide hémorragique traité, où une intervention auprès du patient est nécessaire. Le liquide hémorragique qui a été traité par l'unité de traitement 100 puis transféré dans la poche de transfusion 410 de l'unité de transfusion 400 peut être transfusé au patient. Pour ce faire, il est préférable de déconnecter la poche de transfusion 410 du système de traitement pour la connecter au patient.

La description qui suit précise l'étape E2 de traitement du liquide hémorragique préalablement prélevé auprès d'un patient au cours de l'étape E1. Qu'il y ait une phase préparatoire du circuit de l'unité de traitement 100 comme décrite plus haut ou pas, le premier cycle de traitement débutera lorsque le volume de liquide hémorragique dans le réceptacle de collecte 210 aura atteint un seuil, ce seuil correspondant préférentiellement au volume du bolus que l'on souhaite traiter en un cycle de traitement, ce bolus de liquide hémorragique étant par exemple choisi de l'ordre de 500 ml.

Ainsi, pour traiter des volumes de liquide hémorragique supérieur au volume fixé pour un bolus à traiter au cours d'un cycle de traitement, il conviendra d'effectuer plusieurs cycles de traitement successifs.

Il est à noter que le cycle de traitement pourrait, dans certains cas particuliers, être effectué avec un volume de liquide hémorragique inférieur au volume fixé pour le bolus de traitement, comme par exemple en fin de traitement où il reste moins de liquide hémorragique à traiter, en fin de saignement, ou à n'importe quel moment opportun choisi par le praticien. Il est toutefois à noter qu'il faudra tout de même que le volume de liquide hémorragique à traiter soit supérieur, de préférence au moins deux fois supérieur, au volume mort du circuit de traitement (VmTT), c'est-à-dire le volume compris entre la sortie 140b de la poche de traitement 140 et l'entrée 140a de la poche de traitement 140, c'est-à-dire le volume de la ligne de recirculation 150 et de l'intérieur du dispositif de filtration 113.

Comme déjà indiqué, le cycle de traitement va pouvoir se lancer automatiquement dès que le peson 230 du réceptacle de collecte 210 aura mesuré la quantité cible du bolus. Ce bolus peut par exemple être égal à 500 ml de liquide hémorragique. Ce bolus pourra être mélangé au liquide de dilution stocké dans la poche de traitement 140 (par exemple un volume de 200 ml) pendant la phase de prétraitement et d'amorçage de l'unité de traitement 100. Dans le cas où le volume de dilution n'est pas présent dans la poche de traitement 140, par exemple lorsqu'aucune phase de prétraitement n'aura été lancée ou que le traitement démarre par une phase de filtration dans le dispositif de filtration 113, on pourra introduire directement le volume de liquide de dilution requis dans la ligne de recirculation 150 depuis l'unité de dilution 500,
Pour mélanger le liquide hémorragique du réceptacle de collecte 210 avec le liquide de dilution présent dans la chambre de traitement 140, il convient dans un premier temps de remplir la poche de traitement 140 avec ledit liquide hémorragique. Pour ce faire, les clamps de la valve de régulation 121 de la ligne d'admission 120 et de la valve de régulation 151 de la ligne de recirculation 150 sont ouvertes et la pompe péristaltique 160 est mise en marche en rotation inversée, c'est-à-dire à dire pour entrainer le liquide hémorragique dans le sens inverse du sens de traitement, vers la sortie 140b de la chambre de traitement 140. Lorsque le peson 155 de la poche de traitement 140 détecte que le volume de traitement cible est atteint, la pompe péristaltique 160 est arrêtée. Il est à noter que cette étape de mélange pourrait être effectuée en injectant d'abord le liquide hémorragique dans la poche de traitement 140 ou en réalisant au préalable une filtration et concentration puis en injectant le liquide de dilution dans cette même poche de traitement 140 s'il n'est pas déjà présent.

La phase de traitement du liquide hémorragique a proprement parlé peut ensuite démarrer, avec une filtration et concentration jusqu'à l'obtention de l'hématocrite cible. En général, le taux d'hématocrite recherché est de l'ordre de 45% +/- 5%, mais il pourrait être de l'ordre de 50% +/- 5%, 55% +/- 5%, voire au maximum de 60% +/- 5%. Pour cette phase, on va donc fermer le clamp de la valve de régulation 121 de la ligne d'admission 120 puis ouvrir les clamps de la valve de régulation 152 de la ligne de recirculation 150 et de la valve de régulation 131 de la ligne d'évacuation 130 ; la vanne de commande électrique 320 est également actionnée pour imposer un vide à la chambre d'évacuation 112 à travers l'unité de récupération 300. La pompe péristaltique 160 est alors actionnée dans le sens de traitement, de sorte que le liquide contenu dans la poche de traitement 140 circule depuis la sortie 140b vers l'entrée 111a de la chambre d'admission 111 du dispositif de filtration 110, puis de la sortie 111b de la chambre d'admission 111 du dispositif de filtration 110 jusque vers l'entrée 140a de la poche de traitement 140.

En traversant le dispositif de filtration 110, le liquide hémorragique à traiter est filtré par la membrane de filtration 113 à fibres creuses et est progressivement dépourvu des composés non désirés pour l'autotransfusion (tels que les protéines et autres molécules médicamenteuses qui sont impropres à être transfusées), ceux-ci traversant la membrane de filtration 113 jusque la chambre d'évacuation 112 avant d'être aspirés par la dépression de l'unité de récupération 300 à travers la ligne d'évacuation 130. La recirculation du liquide hémorragique dans la ligne de recirculation 150 à travers successivement le dispositif de filtration 110 et la poche de traitement 140 est effectué jusqu'à l'obtention du taux d'hématocrite cible, ce taux d'hématocrite étant détecté au niveau du capteur d'hématocrite 154.

Une fois que le taux d'hématocrite cible est atteint et que le volume de concentrat (correspondant au liquide hémorragique traité) dans la poche de traitement 140 est suffisant (par exemple supérieur à 100 ml), il peut être envisagé de transférer ce concentrat vers la poche de transfusion 410 de l'unité de transfusion 400.

De préférence mais de manière non obligatoire, il est effectué un rinçage de la ligne de recirculation 150 et de la membrane de filtration 113 du dispositif de filtration 110. Plus spécifiquement, il est envisagé de rincer le volume mort du circuit de filtration (VmCF) qui correspond au volume de circuit compris entre l'unité de transfusion 400 et la poche de traitement 140 passant par le dispositif de filtration 110. A cet égard, on ferme les clamps de la valve de régulation 151 de la ligne de recirculation 150 et de la valve de régulation 131 de la ligne d'évacuation 130 et on ouvre le clamp de la valve de régulation 191 de la ligne de dilution 190. La pompe péristaltique 160 reprend son fonctionnement en sens de traitement pour amener le liquide de dilution de l'unité de dilution 500 en direction du dispositif de filtration 110, ce liquide de dilution étant injecté afin de pousser la colonne de sang du VmCF.

On peut prévoir un capteur optique placé au niveau de l'entrée 140a de la poche de traitement 140 qui permet de détecter la nature du liquide arrivant au niveau de l'entrée 140a. Si un tel capteur est utilisé, il est alors possible de stopper la pompe 160 pour stopper la phase de rinçage dès que le capteur optique détecte la présence du liquide de dilution. Un tel capteur optique permet de commander l'arrêt de la pompe 160 avant que le liquide de lavage ne vienne dans la poche de traitement.

Pour cette phase de rinçage, il est souhaitable que la poche de traitement 140 intègre le dispositif séparateur 141 qui permet de confiner le concentrat dans la chambre de traitement 142, en partie basse de la poche de traitement 140 du côté de la sortie 140b de la poche de traitement 140. L'autre chambre de traitement 141 formée par le dispositif séparateur 141 en partie haute de la poche de traitement 140 du côté de l'entrée 140a de la poche de traitement 140 permet de récupérer le liquide contenu dans le circuit et poussé par le liquide de dilution dans la phase de rinçage.

Il est à noter qu'il est également possible d'effectuer plusieurs phases de concentration successives du même bolus de liquide hémorragique à traiter. Cela permet notamment d'encore mieux supprimer les composés non désirés pour l'autotransfusion, tels que les produits indésirables à la transfusion, de type héparine. De préférence, on effectue une triple concentration pour un même bolus de liquide hémorragique à traiter.

De manière privilégiée, lorsque l'on effectue plusieurs concentrations successives, une fois que le bolus de liquide hémorragique à traiter a atteint le taux d'hématocrite cible après une phase de concentration, on va de nouveau diluer le liquide hémorragique traité pour éliminer les impuretés lors d'une nouvelle concentration,.

Lorsque le transfert est souhaité, après le rinçage optionnel ou directement après la phase de filtration et concentration, et/ou après plusieurs phases de concentration, il convient de fermer les clamps de la valve de régulation 152 de la ligne de recirculation 150 et de la valve de régulation 191 de la ligne de dilution 190 (si cela n'est pas déjà le cas) et d'ouvrir les clamps de la valve de régulation 171 de la ligne de transfusion 170 et de la valve de régulation 151 de la ligne de recirculation 150 (si cela n'est pas déjà le cas). La pompe péristaltique 160 est alors démarrée en sens de traitement et le concentrat est donc transféré depuis poche de traitement 140 jusque dans la poche de transfusion 410 de l'unité de transfusion 400.

Il est à noter que le volume de concentrat présent dans la chambre de traitement 140 pourrait être conservé et donc non transféré immédiatement vers l'unité de transfusion 400. Le concentrat sera alors cumulé avec le concentrat résultant d'un cycle de traitement ultérieur, c'est-à-dire avec un autre bolus.

Une fois que le concentrat a été en tout ou partie transféré à l'unité de transfusion 400, le cycle de traitement est terminé et un autre cycle de traitement peut être enclenché avec un autre bolus de liquide hémorragique.

L'étape E3 de transfusion du liquide hémorragique traité vers le patient peut être faite, en déconnectant notamment la poche de transfusion 410 du système de traitement, et en la connectant au patient. Si une transfusion n'est pas nécessaire immédiatement, la poche de transfusion 410 peut aussi être stockée, une nouvelle poche de transfusion 410 étant connectée au système de traitement, afin de pouvoir récupérer le liquide hémorragique nouvellement traité.

### D. Phase de nettoyage du dispositif de filtration

Lors de filtration sur membrane, une baisse du flux de filtration est généralement constatée tout au long du processus, au cours des cycles de traitement successifs. Ce déclin de la capacité filtrante de la membrane de filtration est dû à plusieurs phénomènes, notamment à l'adsorption et à l'obstruction et colmatage des pores par les composés à filtrer. L'encrassement par adsorption peut représenter des pertes de perméabilité allant jusqu'à 90%, voire jusqu'au blocage total de la filtration.

Le système de traitement proposé, en particulier l'unité de traitement 100 particulière proposée dans le présent document, permet de nettoyer le dispositif de filtration 110 au cours du traitement d'un liquide hémorragique pour un même patient, sans avoir à démonter ledit dispositif de filtration 110 de l'unité de traitement 100 et ainsi avoir un traitement global du liquide hémorragique ne subissant pas ou peu d'interruptions.

Plusieurs types de nettoyage du dispositif de filtration 110 peuvent être envisagés avec le système de traitement proposé, ces types de nettoyage pouvant être réalisés seuls ou en complément les uns des autres.

Le premier type de nettoyage consiste en un rinçage de la chambre d'admission 111 du dispositif de filtration 110 qui est effectué en introduisant du liquide de dilution depuis l'unité de dilution 500 en direction de l'entrée 111a de la chambre d'admission 111 du dispositif de filtration 110. Ce type de rinçage a déjà été présenté plus haut en phase finale du cycle de traitement, avant le transfert effectif du concentrat.

Le deuxième type de nettoyage consiste en un rinçage de la chambre d'évacuation 112 du dispositif de filtration 110 qui est effectué en introduisant du liquide de dilution depuis l'unité de dilution 500 en direction de l'entrée 112a de la chambre d'évacuation 112 du dispositif de filtration 110. Ce rinçage permet de vidanger la chambre d'évacuation 112 du filtrat qui pourrait être encore présent et de l'éliminer vers la poche de récupération 310 via la ligne d'évacuation 130. Pour ce faire, on peut fermer les clamps de la valve de régulation 171 de la ligne de transfusion 170 et de la valve de régulation 151 de la ligne de recirculation 150, et on ouvre les clamps de la valve de régulation 191 de la ligne de dilution 190 et de la valve de régulation 181 de la ligne de nettoyage 180. Il est préférable également que le clamp de la valve de régulation 152 de la ligne de recirculation 150 soit fermé. La pompe péristaltique est alors démarrée pour faire circuler le liquide de dilution dans le sens de traitement de la poche de dilution 500 vers le dispositif de filtration 110.

Le troisième type de nettoyage consiste en un décolmatage de la membrane de filtration 113 du dispositif de filtration 110 par création d'un contre-flux transmembranaire comme on l'a évoqué plus haut. Pour ce faire, on ferme la vanne de commande électrique 320 pour stopper le vide dans l'unité de récupération 300 et on ferme le clamp de la valve de régulation 131 de la ligne d'évacuation 130. On ferme également les clamps de la valve de régulation 171 de la ligne de transfusion 170, de la valve de régulation 151 de la ligne de recirculation 150, et de la valve de régulation 152 de la ligne de recirculation 150, et on ouvre les clamps de la valve de régulation 191 de la ligne de dilution 190 et de la valve de régulation 181 de la ligne de nettoyage 180. Le fait que la ligne d'évacuation 130 soit obstruée, la pression de liquide de nettoyage dans la chambre d'évacuation 112 augmente et décolle ainsi les composés étant fixés sur la membrane de filtration 113. On peut contrôler la pression dans cette chambre d'évacuation 112 en faisant varier la vitesse d'entrainement de la pompe 160 ou en faisant varier le flux passant au niveau de la valve de régulation 181 de la ligne de nettoyage 180. Une fois que les composés ont été décollés de la membrane de filtration 113, un rinçage de la chambre d'évacuation 112 peut être réalisé comme précédemment, en ouvrant le clamp de la valve de régulation 131 de la ligne d'évacuation 130. Il est à noter que le débit de décolmatage, c'est-à-dire le débit de circulation du liquide de nettoyage pour créer le contre-flux transmembranaire est de préférence au moins égal au débit de traitement, c'est-à-dire le débit de circulation du liquide hémorragique dans l'unité de traitement 100. Il a été en effet constaté que cela permettait d'avoir des temps de traitement globaux plus rapides tout en réduisant la perte en globules rouges à chaque cycle. La perte en globules rouges est par exemple uniquement de 5% contre 10% lorsque le débit de décolmatage passe de 1200 ml/min à 600 ml/min. La performance du nettoyage de la fibre en est également améliorée.

Les trois types de nettoyage décrits ci-dessus peuvent être effectués seuls ou en combinaison, les uns après les autres.

Par exemple, il peut être envisagé la séquence de nettoyage en deux temps suivante :
a1. décolmatage de la membrane de filtration 113 du dispositif de filtration 110 selon le troisième type de nettoyage ci-dessus, notamment pour nettoyer la membrane de filtration de l'extérieur vers l'intérieur ; puis
b1. rinçage de la chambre d'admission 111 du dispositif de filtration 110 selon le premier type de nettoyage ci-dessus.

Selon un autre exemple, il peut être envisagé la séquence de nettoyage en trois temps suivante :
a1. rinçage de la chambre d'admission 111 du dispositif de filtration 110 selon le premier type de nettoyage ci-dessus ; puis
b2. décolmatage de la membrane de filtration 113 du dispositif de filtration 110 selon le troisième type de nettoyage ci-dessus, notamment pour nettoyer la membrane de filtration de l'extérieur vers l'intérieur ; puis
c2. rinçage de la chambre d'admission 111 du dispositif de filtration 110 selon le premier type de nettoyage ci-dessus.

Une fois que le dispositif de filtration 110 a été nettoyé, on peut ajuster le volume de dilution présent dans la chambre de traitement 140, ce volume de dilution pouvant être utilisé pour un cycle de traitement ultérieur comme expliqué plus haut en référence à la phase préparatoire du cycle de traitement. Ainsi, après une phase de nettoyage, il convient de fermer le clamp de la valve de régulation 181 de la ligne de nettoyage 180 tout en maintenant fermé le clamp de la valve de régulation 131 de la ligne d'évacuation 130, et on ouvre le clamp de la valve de régulation 152 de la ligne de recirculation 150 tout en continuant de faire circuler le liquide de dilution avec la pompe 160.

REFERENCE BIBLIOGRAPHIQUE : US 4,886,487

## Revendications

1. Système de traitement d'un liquide hémorragique préalablement prélevé chez un patient en vue d'une autotransfusion, incluant une unité de traitement (100) du liquide hémorragique, ladite unité de traitement (100) comprenant :
- un dispositif de filtration (110) comprenant une membrane de filtration (113) pour filtration tangentielle agencée dans un boitier (114) de manière à séparer une chambre d'admission (111) d'une chambre d'évacuation (112), la chambre d'admission (111) et la chambre d'évacuation (112) ayant chacune une entrée (111a ;112a) et une sortie (111b ; 112b) de fluides ;
- une poche de traitement (140) ayant une entrée (140a) et une sortie (140b) reliées fluidiquement par une ligne de recirculation (150) à la sortie (111b) et à l'entrée (111a) de la chambre d'admission (111) du dispositif de filtration (110), respectivement, permettant une circulation du liquide hémorragique dans la ligne de recirculation (150) dans un sens allant de la sortie (140b) de la poche de traitement (140) vers l'entrée (140a) de la poche de traitement (140) à travers la chambre d'admission (111) du dispositif de filtration (110) ;
- une ligne d'admission (120) reliée fluidiquement à la ligne de recirculation (150) entre la sortie (140b) de la poche de traitement (140) et l'entrée (111a) de la chambre d'admission (111) du dispositif de filtration (110) permettant d'alimenter l'unité de traitement (100) avec le liquide hémorragique prélevé en vue d'une filtration à travers la membrane de filtration (113) du dispositif de filtration (110) afin de retirer du liquide hémorragique un filtrat comprenant des composés non désirés pour l'autotransfusion ;
- une ligne de transfusion (170) reliée fluidiquement à la ligne de recirculation (150) entre la sortie (140b) de la poche de traitement (140) et l'entrée (111a) de la chambre d'admission (111) du dispositif de filtration (110) permettant de récupérer le liquide hémorragique traité contenu dans ladite poche de traitement (140) ;
- une ligne d'évacuation (130) reliée fluidiquement à la sortie (112b) de la chambre d'évacuation (112) du dispositif de filtration (110) de sorte à évacuer le filtrat ayant traversé la membrane de filtration (113) depuis la chambre d'admission (111) ;
**caractérisé en ce que** l'unité de traitement comprend en outre
- un premier organe de régulation de flux (151) agencé pour une régulation de flux dans la ligne de recirculation (150) à la sortie (140b) de la poche de traitement (140), et
- une ligne de dilution (190) destinée à amener un liquide de dilution dans l'unité de traitement (100), la ligne de dilution (190) étant reliée fluidiquement à la ligne de recirculation (150) à une position entre la sortie (140b) de la poche de traitement (140) et l'entrée (111a) de la chambre d'admission (111) du dispositif de filtration (110).

2. Système de traitement selon la revendication 1, dans lequel un capteur optique est agencé au niveau de l'entrée (140a) de la poche de traitement (140) pour détecter la nature du liquide arrivant au niveau de l'entrée (140a).

3. Système de traitement selon l'une quelconque des revendications 1 et 2, dans lequel la poche de traitement (140) comprend un dispositif séparateur (141) pouvant être actionné pour séparer la poche de traitement (140) en une première chambre de traitement (142) du côté de l'entrée (140a) de la poche de traitement (140) et une deuxième chambre de traitement (143) du côté de la sortie (140b) de la poche de traitement (140).

4. Système de traitement selon l'une quelconque des revendications 1 à 3, dans lequel l'unité de traitement (100) comprend un deuxième organe de régulation de flux (191) agencé pour une régulation de flux dans la ligne de dilution (190), et un troisième organe de régulation de flux (152) agencé pour une régulation de flux dans la ligne de recirculation (150) à l'entrée (111a) de la chambre d'admission (111) du dispositif de filtration (110).

5. Système de traitement selon l'une quelconque des revendications 1 à 4, dans lequel la poche de traitement (140) a une forme sensiblement parallélépipédique avec l'entrée (140a) et la sortie (140b) de part et d'autre de la poche de traitement selon une diagonale, la poche de traitement présentant en outre une cavité interne ayant une forme rétrécie du côté de la sortie (140b).

6. Système de traitement selon l'une quelconque des revendications 1 à 5, dans lequel la membrane de filtration (113) du dispositif de filtration (110) est une membrane de filtration (113) à fibres creuses, lesdites fibres creuses formant la membrane de filtration (113) s'étendant longitudinalement dans le boitier (114), dans lequel la membrane de filtration (113) à fibres creuses du dispositif de filtration (110) comprend de préférence des fibres creuses formées à partir d'un mélange de polyester sulfone et de polyvinyle pyrrolidone.

7. Système de traitement selon l'une quelconque des revendications 1 à 6, dans lequel la membrane de filtration (113) du dispositif de filtration (110) a une porosité globale comprise entre 0,1 µm et 1 µm, de préférence de l'ordre de 0,6 µm, et/ou dans lequel la membrane de filtration (113) du dispositif de filtration (110) a une surface de filtration globale comprise entre 0,1 m² et 1 m², et de préférence comprise entre 0,2 m² et 0,6 m².

8. Système de traitement selon l'une quelconque des revendications 1 à 7, comprenant au moins une pompe péristaltique (160) agencée de manière à faire circuler le liquide hémorragique dans la ligne de recirculation (150) dans un sens allant de la sortie (140b) de la poche de traitement (140) vers l'entrée (140a) de la poche de traitement (140) à travers la chambre d'admission (111) du dispositif de filtration (110).

9. Système de traitement selon l'une quelconque des revendications 1 à 8, dans lequel l'unité de traitement (100) comprend un gabarit (101) permettant une fixation de la ligne d'admission (120), la ligne d'évacuation (130), la ligne de recirculation (150), la ligne de dilution (190), et la ligne de transfusion (170),
le système de traitement comprenant de préférence une unité de support (10), le gabarit (101) de l'unité de traitement (100) ayant une forme en détrompeur permettant de coupler l'unité de traitement (100) à l'unité de support (10) selon un unique positionnement,
dans lequel l'unité de support (10) forme de préférence un plan de support horizontal, le dispositif de filtration (110) de l'unité de traitement (100) étant destiné à être couplé à l'unité de support (10) de sorte que des fibres creuses de la membrane de filtration (113) s'étendent selon une direction non comprise dans le plan de support horizontal.

10. Système de traitement selon l'une quelconque des revendications 1 à 9, comprenant une unité de transfusion (400), ladite unité de transfusion (400) comprenant une poche de transfusion (410) ayant une entrée destinée à être reliée à la ligne de transfusion (170) afin de collecter le liquide hémorragique traité en provenance de la poche de traitement (140) avant une transfusion au patient.

11. Système de traitement selon l'une quelconque des revendications 1 à 10, comprenant une unité de récupération (300) du filtrat, ladite unité de récupération (300) comprenant une poche de récupération (310) ayant une entrée (310a) destinée à être reliée fluidiquement à la ligne d'évacuation (130),
dans lequel ladite poche de récupération (310) est en outre destinée à être couplée à un dispositif de mise en dépression de la poche de récupération (310) de manière à faire circuler le filtrat de la chambre d'évacuation (112) du dispositif de filtration (110) vers la poche de récupération (310) à travers la ligne d'évacuation (130), et/ou
dans lequel ladite poche de récupération (310) est en outre agencée par rapport au dispositif de filtration (110) de l'unité de traitement (100) pour créer une dépression de la poche de récupération (310) par rapport au dispositif de filtration (110) de manière à faire circuler le filtrat de la chambre d'évacuation (112) du dispositif de filtration (110) vers la poche de récupération (30) à travers la ligne d'évacuation (130).

12. Système de traitement selon l'une quelconque des revendications 1 à 11, comprenant une unité de collecte (200) du liquide hémorragique comprenant un réceptacle de collecte (210) du liquide hémorragique préalablement prélevé chez le patient, ledit réceptacle de collecte (210) ayant une sortie (210b) reliée fluidiquement à la ligne d'admission (120), ledit réceptacle de collecte (210) intégrant de préférence un dispositif de préfiltration (220) permettant de faire une préfiltration du liquide hémorragique avant d'être transmis dans l'unité de traitement (100), le système de traitement comprenant de préférence en outre un dispositif de préfiltration supplémentaire (122) placé dans la ligne d'admission (120).

13. Procédé d'utilisation du système de traitement selon l'une quelconque des revendications 1 à 12 pour le traitement de liquide hémorragique préalablement prélevé chez un patient en vue d'une autotransfusion ultérieure, dans lequel le système de traitement n'est pas relié au patient, **caractérisé en ce que**, après un traitement partiel ou total du liquide hémorragique avec le dispositif de filtration (110), on injecte un liquide de dilution depuis la ligne de dilution (190) dans la ligne de circulation afin de traverser le dispositif de filtration (110).

14. Procédé selon la revendication 13, dans lequel on effectue un nettoyage de la membrane de filtration (113) par rinçage, le rinçage étant opéré en obstruant la sortie (140b) de la poche de traitement (140) au niveau du premier organe de régulation de flux (151), en obstruant la ligne d'évacuation (130) au niveau de la sortie (112b) de la chambre d'évacuation (112) du dispositif de filtration (110), et en injectant le liquide de nettoyage dans la chambre d'admission (111) depuis la ligne de dilution (190), le rinçage étant de préférence arrêté dès que le capteur optique détecte la présence du liquide de dilution.

15. Procédé selon la revendication 14, dans lequel avant le rinçage, on commande le dispositif séparateur (141) pour isoler le liquide hémorragique traité dans la deuxième chambre de traitement (143).

16. Procédé selon l'une quelconque des revendications14 et 15, dans lequel on effectue une dilution du liquide hémorragique contenu dans le système de traitement, la dilution étant opérée en obstruant la sortie (140b) de la poche de traitement (140) au niveau du premier organe de régulation de flux (151), puis en injectant le liquide de nettoyage dans la chambre d'admission (111) depuis la ligne de dilution (190), puis en isolant le liquide hémorragique traité présent dans la poche de traitement (140) lorsque le liquide présent dans la ligne de circulation (150) a un taux d'hématocrite inférieur à une valeur seuil.

## Patentansprüche

1. System zur Behandlung einer vorhergehend bei einem Patienten entnommenen hämorrhagischen Flüssigkeit in Hinblick auf eine Autotransfusion, das eine Einheit (100) zur Behandlung der hämorrhagischen Flüssigkeit umfasst, wobei die Behandlungseinheit (100) umfasst:
- eine Filtrationsvorrichtung (110), die eine Filtrationsmembran (113) zur Tangentialflussfiltration umfasst, die derart in einem Gehäuse (114) angeordnet ist, dass sie eine Einlasskammer (111) von einer Ablasskammer (112) trennt, wobei die Einlasskammer (111) und die Ablasskammer (112) jeweils einen Eingang (111a; 112a) und einen Ausgang (111b; 112b) für Fluide aufweisen;
- einen Behandlungsbeutel (140), der einen Eingang (140a) und einen Ausgang (140b) aufweist, die durch eine Rückführungsleitung (150) fluidisch mit dem Ausgang (111b) beziehungsweise mit dem Eingang (111a) der Einlasskammer (111) der Filtrationsvorrichtung (110) verbunden sind und einen Umlauf der hämorrhagischen Flüssigkeit in der Rückführungsleitung (150) in einer Richtung ermöglichen, die von dem Ausgang (140b) des Behandlungsbeutels (140) hin zum Eingang (140a) des Behandlungsbeutels (140) durch die Einlasskammer (111) der Filtrationsvorrichtung (110) verläuft;
- eine Einlassleitung (120), die fluidisch mit der Rückführungsleitung (150) zwischen dem Ausgang (140b) des Behandlungsbeutels (140) und dem Eingang (111a) der Einlasskammer (111) der Fitrationsvorrichtung (110) verbunden ist und das Versorgen der Behandlungseinheit (100) mit der entnommenen hämorrhagischen Flüssigkeit im Hinblick auf eine Filtration durch die Filtrationsmembran (113) der Filtrationsvorrichtung (110) ermöglicht, um von der hämorrhagischen Flüssigkeit ein Filtrat zu entfernen, das für die Autotransfusion unerwünschte Verbindungen umfasst;
- eine Transfusionsleitung (170), die fluidisch mit der Rückführungsleitung (150) zwischen dem Ausgang (140b) des Behandlungsbeutels (140) und dem Eingang (111a) der Einlasskammer (111) der Filtrationsvorrichtung (110) verbunden ist und das Wiedergewinnen der behandelten hämorrhagischen Flüssigkeit ermöglicht, die in dem Behandlungsbeutel (140) enthalten ist;
- eine Ablassleitung (130), die derart fluidisch mit dem Ausgang (112b) der Ablasskammer (112) der Filtrationsvorrichtung (110) verbunden ist, dass das Filtrat, das die Filtrationsmembran (113) ausgehend von der Einlasskammer (111) durchquert hat, abgelassen wird;
**dadurch gekennzeichnet, dass** die Behandlungseinheit ferner umfasst:
- ein erstes Flussregelungsorgan (151), das für eine Flussregelung in der Rückführungsleitung (150) am Ausgang (140b) des Behandlungsbeutels (140) angeordnet ist, und
- eine Verdünnungsleitung (190), die dazu ausgestaltet ist, eine Verdünnungsflüssigkeit in die Behandlungseinheit (100) zu führen, wobei die Verdünnungsleitung (190) fluidisch mit der Rückführungsleitung (150) an einer Position zwischen dem Ausgang (140b) des Behandlungsbeutels (140) und dem Eingang (111a) der Einlasskammer (111) der Filtrationsvorrichtung (110) verbunden ist.

2. Behandlungssystem nach Anspruch 1, wobei ein optischer Sensor im Bereich des Eingangs (140a) des Behandlungsbeutels (140) angeordnet ist, um die Beschaffenheit der Flüssigkeit zu erkennen, die im Bereich des Eingangs (140a) eintrifft.

3. Behandlungssystem nach einem der Ansprüche 1 und 2, wobei der Behandlungsbeutel (140) eine Trennungsvorrichtung (141) umfasst, die betätigt werden kann, um den Behandlungsbeutel (140) in eine erste Behandlungskammer (142) auf der Seite des Eingangs (140a) des Behandlungsbeutels (140) und eine zweite Behandlungskammer (143) auf der Seite des Ausgangs (140b) des Behandlungsbeutels (140) zu trennen.

4. Behandlungssystem nach einem der Ansprüche 1 bis 3, wobei die Behandlungseinheit (100) ein zweites Flussregelungsorgan (191), das für eine Flussregelung in der Verdünnungsleitung (190) angeordnet ist, und ein drittes Flussregelungsorgan (152) umfasst, das für eine Flussregelung in der Rückführungsleitung (150) am Eingang (111a) der Einlasskammer (111) der Filtrationsvorrichtung (110) angeordnet ist.

5. Behandlungssystem nach einem der Ansprüche 1 bis 4, wobei der Behandlungsbeutel (140) eine im Wesentlichen parallelepipedische Form mit dem Eingang (140a) und dem Ausgang (140b) auf beiden Seiten des Behandlungsbeutels entlang einer Diagonalen aufweist, wobei der Behandlungsbeutel ferner einen inneren Hohlraum aufweist, der auf der Seite des Ausgangs (140b) eine verjüngte Form aufweist.

6. Behandlungssystem nach einem der Ansprüche 1 bis 5, wobei die Filtrationsmembran (113) der Filtrationsvorrichtung (110) eine Filtrationsmembran (113) mit Hohlfasern ist, wobei die Hohlfasern die Filtrationsmembran (113) sich in Längsrichtung in dem Gehäuse (114) erstreckend bilden, wobei die Fitrationsmembran (113) mit Hohlfasern der Filtrationsvorrichtung (110) vorzugsweise Hohlfasern umfasst, die ausgehend von einem Gemisch aus Polyestersulfon und Polyvinylpyrrolidon gebildet sind.

7. Behandlungssystem nach einem der Ansprüche 1 bis 6, wobei die Filtrationsmembran (113) der Filtrationsvorrichtung (110) eine Gesamtporosität von zwischen 0,1 µm und 1 µm, vorzugsweise in der Größenordnung von 0,6 µm, aufweist und/oder wobei die Filtrationsmembran (113) der Filtrationsvorrichtung (110) eine Gesamtfiltrationsoberfläche von zwischen 0,1 m² und 1 m² und vorzugsweise von zwischen 0,2 m² und 0,6 m² aufweist.

8. Behandlungssystem nach einem der Ansprüche 1 bis 7, das mindestens eine Peristaltikpumpe (160) umfasst, die derart angeordnet ist, dass die hämorrhagische Flüssigkeit in der Rückführungsleitung (150) in einer Richtung in Umlauf gebracht wird, die vom Ausgang (140b) des Behandlungsbeutels (140) hin zum Eingang (140a) des Behandlungsbeutels (140) durch die Einlasskammer (111) der Filtrationsvorrichtung (110) verläuft.

9. Behandlungssystem nach einem der Ansprüche 1 bis 8, wobei die Behandlungseinheit (100) ein Profil (101) umfasst, das eine Befestigung der Einlassleitung (120), der Ablassleitung (130), der Rückführungsleitung (150), der Verdünnungsleitung (190) und der Transfusionsleitung (170) ermöglicht,
wobei das Behandlungssystem vorzugsweise eine Stützeinheit (10) umfasst, wobei das Profil (101) der Behandlungseinheit (100) eine unverwechselbare Form aufweist, die das Koppeln der Behandlungseinheit (100) an die Stützeinheit (10) gemäß einer einzigen Positionierung ermöglicht,
wobei die Stützeinheit (10) vorzugsweise eine horizontale Stützebene bildet, wobei die Filtrationsvorrichtung (110) der Behandlungseinheit (100) dazu gestaltet ist, derart an die Stützeinheit (10) gekoppelt zu sein, dass Hohlfasern der Filtrationsmembran (113) sich entlang einer Richtung erstrecken, die nicht in der horizontalen Stützebene enthalten ist.

10. Behandlungssystem nach einem der Ansprüche 1 bis 9, das eine Transfusionseinheit (400) umfasst, wobei die Transfusionseinheit (400) einen Transfusionsbeutel (410) umfasst, der einen Eingang aufweist, der dazu bestimmt ist, mit der Transfusionsleitung (170) verbunden zu sein, um die von dem Behandlungsbeutel (140) stammende behandelte hämorrhagische Flüssigkeit vor einer Transfusion an den Patienten zu sammeln.

11. Behandlungssystem nach einem der Ansprüche 1 bis 10, das eine Einheit (300) zur Rückgewinnung des Filtrats umfasst, wobei die Rückgewinnungseinheit (300) einen Rückgewinnungsbeutel (310) umfasst, der einen Eingang (310a) aufweist, der dazu bestimmt ist, fluidisch mit der Ablassleitung (130) verbunden zu sein,
wobei der Rückgewinnungsbeutel (310) ferner dazu bestimmt ist, an eine Vorrichtung zur Erzeugung von Unterdruck in dem Rückgewinnungsbeutel (310) gekoppelt zu sein, derart dass das Filtrat von der Ablasskammer (112) der Filtrationsvorrichtung (110) durch die Ablassleitung (130) hin zum Rückgewinnungsbeutel (310) in Umlauf gebracht wird, und/oder
wobei der Rückgewinnungsbeutel (310) ferner in Bezug auf die Filtrationsvorrichtung (110) der Behandlungseinheit (100) angeordnet ist, um einen Unterdruck in dem Rückgewinnungsbeutel (310) in Bezug auf die Filtrationsvorrichtung (110) zu erzeugen, derart dass das Filtrat der Ablasskammer (112) der Filtrationsvorrichtung (110) durch die Ablassleitung (130) hin zum Rückgewinnungsbeutel (30) in Umlauf gebracht wird.

12. Behandlungssystem nach einem der Ansprüche 1 bis 11, das eine Einheit (200) zur Sammlung der hämorrhagischen Flüssigkeit umfasst, die einen Behälter (210) zur Sammlung der vorhergehend bei dem Patienten entnommenen hämorrhagischen Flüssigkeit umfasst, wobei der Sammelbehälter (210) einen Ausgang (210b) aufweist, der fluidisch mit der Einlassleitung (120) verbunden ist, wobei der Sammelbehälter (210) vorzugsweise eine Vorfiltrationsvorrichtung (220) aufnimmt, die das Durchführen einer Vorfiltration der hämorrhagischen Flüssigkeit ermöglicht, bevor sie in die Behandlungseinheit (100) übertragen wird, wobei das Behandlungssystem vorzugsweise ferner eine zusätzliche Vorfiltrationsvorrichtung (122) umfasst, die in der Einlassleitung (120) platziert ist.

13. Verfahren zur Verwendung des Behandlungssystems nach einem der Ansprüche 1 bis 12 für die Behandlung von vorhergehend bei einem Patienten entnommener hämorrhagischer Flüssigkeit im Hinblick auf eine spätere Autotransfusion, wobei das Behandlungssystem nicht mit dem Patienten verbunden ist, **dadurch gekennzeichnet, dass** nach einer partiellen oder vollständigen Behandlung der hämorrhagischen Flüssigkeit mit der Filtrationsvorrichtung (110) eine Verdünnungsflüssigkeit ausgehend von der Verdünnungsleitung (190) in die Umlaufleitung eingespritzt wird, um die Filtrationsvorrichtung (110) zu durchqueren.

14. Verfahren nach Anspruch 13, wobei eine Reinigung der Filtrationsmembran (113) durch Spülung durchgeführt wird, wobei die Spülung durch Versperren des Ausgangs (140b) des Behandlungsbeutels (140) im Bereich des ersten Flussregelungsorgans (151), durch Versperren der Ablassleitung (130) im Bereich des Ausgangs (112b) der Ablasskammer (112) der Filtrationsvorrichtung (110) und durch Einspritzen der Reinigungsflüssigkeit in die Einlasskammer (111) ausgehend von der Verdünnungsleitung (190) durchgeführt wird, wobei die Spülung vorzugsweise angehalten wird, sobald der optische Sensor das Vorhandensein der Verdünnungsflüssigkeit erkennt.

15. Verfahren nach Anspruch 14, wobei vor der Spülung die Trennungsvorrichtung (141) gesteuert wird, um die in der zweiten Behandlungskammer (143) behandelte hämorrhagische Flüssigkeit zu isolieren.

16. Verfahren nach einem der Ansprüche 14 und 15, wobei eine Verdünnung der in dem Behandlungssystem enthaltenen hämorrhagischen Flüssigkeit durchgeführt wird, wobei die Verdünnung durch Versperren des Ausgangs (140b) des Behandlungsbeutels (140) im Bereich des ersten Flussregelungsorgans (151), dann durch Einspritzen der Reinigungsflüssigkeit in die Einlasskammer (111) ausgehend von der Verdünnungsleitung (190), dann durch Isolieren der in dem Behandlungsbeutel (140) vorhandenen behandelten hämorrhagischen Flüssigkeit vorgenommen wird, wenn die in der Umlaufleitung (150) vorhandene hämorrhagische Flüssigkeit einen Hämatokritgehalt aufweist, der niedriger als ein Schwellenwert ist.

## Claims

1. A treatment system for treating haemorrhagic fluid previously taken from a patient for the purpose of autotransfusion, comprising a treatment unit (100) for treating haemorrhagic fluid, said treatment unit (100) comprising:
- a filtration device (110) comprising a filtration membrane (113) for tangential filtration arranged in a housing (114) so as to separate an intake chamber (111) from a discharge chamber (112), the intake chamber (111) and the discharge chamber (112) each having an inlet (111a; 112a) and an outlet (111b; 112b) for fluids;
- a treatment pouch (140) having an inlet (140a) and an outlet (140b) fluidically connected by a recirculation line (150) to the outlet (111b) and to the inlet (111a) of the intake chamber (111) of the filtration device (110), respectively, allowing haemorrhagic fluid to circulate in the recirculation line (150) in a direction going from the outlet (140b) of the treatment pouch (140) to the inlet (140a) of the treatment pouch (140) through the intake chamber (111) of the filtration device (110);
- an intake line (120) fluidically connected to the recirculation line (150) between the outlet (140b) of the treatment pouch (140) and the inlet (111a) of the intake chamber (111) of the filtration device (110) making it possible to supply the treatment unit (100) with haemorrhagic fluid taken for the purpose of filtration through the filtration membrane (113) of the filtration device (110) in order to remove from the haemorrhagic fluid a filtrate comprising compounds undesired for autotransfusion;
- a transfusion line (170) fluidically connected to the recirculation line (150) between the outlet (140b) of the treatment pouch (140) and the inlet (111a) of the intake chamber (111) of the filtration device (110) making it possible to recover treated haemorrhagic fluid contained in said treatment pouch (140);
- a discharge line (130) fluidically connected to the outlet (112b) of the discharge chamber (112) of the filtration device (110) so as to discharge the filtrate having passed through the filtration membrane (113) from the intake chamber (111);
**characterised in that** the treatment unit further comprises
- a first flow regulation member (151) arranged to regulate the flow in the recirculation line (150) at the outlet (140b) of the treatment pouch (140), and
- a dilution line (190) intended to convey dilution fluid into the treatment unit (100), the dilution line (190) being fluidically connected to the recirculation line (150) at a position between the outlet (140b) of the treatment pouch (140) and the inlet (111a) of the intake chamber (111) of the filtration device (110).

2. The treatment system of claim 1, wherein an optical sensor is arranged at the level of the inlet (140a) of the treatment pouch (140) to detect the nature of the fluid arriving at the level of the inlet (140a).

3. The treatment system of any one of claims 1 and 2, wherein the treatment pouch (140) comprises a separating device (141) being able to be actuated to separate the treatment pouch (140) into a first treatment chamber (142) on the side of the inlet (140a) of the treatment pouch (140) and a second treatment chamber (143) on the side of the outlet (140b) of the treatment pouch (140).

4. The treatment system of any one of claims 1 to 3, wherein the treatment unit (100) comprises a second flow regulation member (191) arranged to regulate the flow in the dilution line (190), and a third flow regulation member (152) arranged to regulate the flow in the recirculation line (150) at the inlet (111a) of the intake chamber (111) of the filtration device (110).

5. The treatment system of any one of claims 1 to 4, wherein the treatment pouch (140) has a substantially parallelepiped shape with the inlet (140a) and the outlet (140b) on either side of the treatment pouch along a diagonal, the treatment pouch further having an inner cavity having a tapering shape on the side of the outlet (140b).

6. The treatment system of any one of claims 1 to 5, wherein the filtration membrane (113) of the filtration device (110) is a filtration membrane (113) with hollow fibres, said hollow fibres forming the filtration membrane (113) extending longitudinally in the housing (114), wherein the filtration membrane (113) with hollow fibres of the filtration device (110) preferably comprises hollow fibres formed from a mixture of polyester sulfone and polyvinyl pyrrolidone.

7. The treatment system of any one of claims 1 to 6, wherein the filtration membrane (113) of the filtration device (110) has an overall porosity comprised between 0.1 µm and 1 µm, preferably of the order of 0.6 µm, and/or wherein the filtration membrane (113) of the filtration device (110) has an overall filtration surface area comprised between 0.1 m² and 1 m², and preferably comprised between 0.2 m² and 0.6 m².

8. The treatment system of any one of claims 1 to 7, comprising at least one peristaltic pump (160) arranged so as to make the haemorrhagic fluid circulate in the recirculation line (150) in a direction going from the outlet (140b) of the treatment pouch (140) to the inlet (140a) of the treatment pouch (140) through the intake chamber (111) of the filtration device (110).

9. The treatment system of any one of claims 1 to 8, wherein the treatment unit (100) comprises a template (101) enabling fixation of the intake line (120), the discharge line (130), the recirculation line (150), the dilution line (190), and the transfusion line (170), the treatment system preferably comprising a support unit (10), the template (101) of the treatment unit (100) having a mistake-proofing shape making it possible to couple the treatment unit (100) to the support unit (10) according to a unique positioning, wherein the support unit (10) preferably forms a horizontal support plane, the filtration device (110) of the treatment unit (100) being intended to be coupled to the support unit (10) such that the hollow fibres of the filtration membrane (113) extend along a direction not comprised in the horizontal support plane.

10. The treatment system of any one of claims 1 to 9, comprising a transfusion unit (400), said transfusion unit (400) comprising a transfusion pouch (410) having an inlet intended to be connected to the transfusion line (170) in order to collect treated haemorrhagic fluid coming from the treatment pouch (140) before transfusion to the patient.

11. The treatment system of any one of claims 1 to 10, comprising a recovery unit (300) for recovering the filtrate, said recovery unit (300) comprising a recovery pouch (310) having an inlet (310a) intended to be fluidically connected to the discharge line (130), wherein said recovery pouch (310) is further intended to be coupled to a device for depressurising the recovery pouch (310) so as to make the filtrate circulate from the discharge chamber (112) of the filtration device (110) to the recovery pouch (310) through the discharge line (130), and/or wherein said recovery pouch (310) is further arranged with respect to the filtration device (110) of the treatment unit (100) to create a vacuum pressure in the recovery pouch (310) with respect to the filtration device (110) so as to make the filtrate circulate from the discharge chamber (112) of the filtration device (110) to the recovery pouch (30) through the discharge line (130).

12. The treatment system of any one of claims 1 to 11, comprising a collection unit (200) for collecting haemorrhagic fluid comprising a collection receptacle (210) for collecting haemorrhagic fluid previously taken from the patient, said collection receptacle (210) having an outlet (210b) fluidically connected to the intake line (120), said collection receptacle (210) preferably integrating a prefiltration device (220) making it possible to carry out a prefiltration of the haemorrhagic fluid before being transmitted into the treatment unit (100), the treatment system preferably further comprising an additional prefiltration device (122) positioned in the intake line (120).

13. A method for using the treatment system of any one of claims 1 to 12 for the treatment of haemorrhagic fluid previously taken from a patient for the purpose of later autotransfusion, wherein the treatment system is not coupled to the patient, **characterised in that**, after partial or total treatment of the haemorrhagic fluid with the filtration device (110), a dilution fluid is injected from the dilution line (190) into the circulation line in order to pass through the filtration device (110).

14. The method of claim 13, wherein cleaning of the filtration membrane (113) is carried out by rinsing, the rinsing being carried out by obstructing the outlet (140b) of the treatment pouch (140) at the level of the first flow regulation member (151), by obstructing the discharge line (130) at the level of the outlet (112b) of the discharge chamber (112) of the filtration device (110), and by injecting cleaning fluid into the intake chamber (111) from the dilution line (190), wherein rinsing is preferably stopped as soon as the optical sensor detects the presence of the dilution fluid.

15. The method of claim 14, wherein, before rinsing, the separating device (141) is controlled to isolate the treated haemorrhagic fluid in the second treatment chamber (143).

16. The method of any one of claims 14 and 15, wherein a dilution of the haemorrhagic fluid contained in the treatment system is carried out, the dilution being performed by obstructing the outlet (140b) of the treatment pouch (140) at the level of the first flow regulation member (151), then by injecting cleaning fluid into the intake chamber (111) from the dilution line (190), then by isolating the treated haemorrhagic fluid present in the treatment pouch (140) when the fluid present in the circulation line (150) has a haematocrit level below a threshold value.
